# EUROPEAN PATENT SPECIFICATION

(11) **EP 2 140 877 B1**
(45) Date of publication and mention of the grant of the patent: **21.01.2015**
(21) Application number: 08733851.3
(22) Date of filing: 28.03.2008
(51) Int. Cl.: A61K 35/68, A61K 39/00, A61K 39/012, A61K 39/145

(54) **NEW USE OF COCCIDIA**
NEUE VERWENDUNG VON COCCIDIA
NOUVELLE UTILISATION DE COCCIDIES

(30) Priority: 29.03.2007 CN 200710064924
(43) Date of publication of application: 06.01.2010
(73) Proprietor: China Agricultural University, Haidian District Beijing 100193, P.R. (CN)
(72) Inventor: SUO, Xun, Beijing 100094 (CN); WANG, Xiaojia, Beijing 100094 (CN); LIU, Xianyong, Beijing 100094 (CN); SHI, Tuanyuan, Beijing 100094 (CN); HAO, Lili, Beijing 100094 (CN); YAN, Wenchao, Beijing 100094 (CN); WANG, Hongyan, Beijing 100094 (CN); LI, Jianan, Beijing 100094 (CN)
(74) Representative: Zardi, Marco
(86) International application number: PCT/CN2008/000621
(87) International publication number: WO 2008/119254

(56) References cited:
- WO-A1-2004/052393
- CN-A- 1 735 429
- CN-A- 100 998 866
- CN-A- 101 024 076
- US-A- 5 976 553
- SHIRLEY MARTIN W ET AL: "The biology of avian Eimeria with an emphasis on their control by vaccination" ADVANCES IN PARASITOLOGY, ACADEMIC PRESS, LONDON, GB, vol. 60, 1 January 2005 (2005-01-01), pages 285-330, XP009111817 ISSN: 0065-308X
- HAO ET AL: "Transient transfection of Eimeria tenella using yellow or red fluorescent protein as a marker" MOLECULAR AND BIOCHEMICAL PARASITOLOGY, ELSEVIER SCIENCE PUBLISHERS, AMSTERDAM, NL, vol. 153, no. 2, 24 February 2007 (2007-02-24), pages 213-215, XP022051425 ISSN: 0166-6851
- KELLEHER MICHELLE ET AL: "Transient expression of beta-galactosidase in differentiating sporozoites of Eimeria tenella" MOLECULAR AND BIOCHEMICAL PARASITOLOGY, ELSEVIER SCIENCE PUBLISHERS, AMSTERDAM, NL, vol. 97, no. 1-2, 30 November 1998 (1998-11-30), pages 21-31, XP009130682 ISSN: 0166-6851 [retrieved on 1998-12-15]
- CHAREST H ET AL: "Recombinant Attenuated Toxoplasma gindii Expressing the Plasmodium yoelii Circumsporozoite Protein Provide High Effective Priming for CD8 T Cell-Dependent Protective Immunity Against Malaria" JOURNAL OF IMMUNOLOGY, AMERICAN ASSOCIATION OF IMMUNOLOGISTS, US, vol. 165, 1 January 2000 (2000-01-01), pages 2084-2092, XP003003660 ISSN: 0022-1767
- DONALD ROBERT G K ET AL: "Stable molecular transformation of Toxoplasma gondii: A selectable dihydrofolate reductase-thymidylate synthase marker based on drug-resistance mutations in malaria" PROCEEDINGS OF THE NATIONAL ACADEMY OF SCIENCES OF USA, NATIONAL ACADEMY OF SCIENCE, WASHINGTON, DC, US, vol. 90, no. 24, 15 December 1993 (1993-12-15), pages 11703-11707, XP009130690 ISSN: 0027-8424
- HONG Y H ET AL: "Changes in immune-related gene expression and intestinal lymphocyte subpopulations following Eimeria maxima infection of chickens" VETERINARY IMMUNOLOGY AND IMMUNOPATHOLOGY, AMSTERDAM, NL, vol. 114, no. 3-4, 15 December 2006 (2006-12-15), pages 259-272, XP024998897 ISSN: 0165-2427 [retrieved on 2006-12-15]
- LIU X ET AL: "Restriction enzyme-mediated transfection improved transfection efficiency in vitro in Apicomplexan parasite Eimeria tenella" MOLECULAR AND BIOCHEMICAL PARASITOLOGY, ELSEVIER SCIENCE PUBLISHERS, AMSTERDAM, NL, vol. 161, no. 1, 1 September 2008 (2008-09-01), pages 72-75, XP023315669 ISSN: 0166-6851 [retrieved on 2008-06-20]
- YAN W ET AL: "Stable transfection of Eimeria tenella: Constitutive expression of the YFP-YFP molecule throughout the life cycle" INTERNATIONAL JOURNAL OF PARASITOLOGY, PERGAMON PRESS, GB, vol. 39, no. 1, 1 January 2009 (2009-01-01), pages 109-117, XP025800541 ISSN: 0020-7519 [retrieved on 2008-07-27]
- GERILETU ET AL.: 'Construction of two DNA vaccines chicken E. tenella and their expressions in chicken muscles' JOUR. OF NORTHWEST SCI.-TECH. UNIV. OF AGRIC. AND FOR. (NAT. SCI. ED.) vol. 33, no. 112, December 2005, pages 34 - 38, 42, XP009130599

### SEQUENCE LIST

SEQUENCE LIST
<110> China Agricultural University
<120> New use of coccidia
<130>
<150> CN200710064924
   <151> 2007-03-29
<160> 6
<170> PatentIn version 3.3
<210> 1
   <211> 14355
   <212> DNA
   <213> artificial sequence
<400> 1
<210> 2
   <211> 8654
   <212> DNA
   <213> artificial sequence
<400> 2
   tagttattaa tagtaatcaa ttacggggtc attagttcat agcccatata tggagttccg     60
   cgttacataa cttacggtaa atggcccgcc tggctgaccg cccaacgacc cccgcccatt    120
   gacgtcaata atgacgtatg ttcccatagt aacgccaata gggactttcc attgacgtca    180
   atgggtggag tatttacggt aaactgccca cttggcagta catcaagtgt atcatatgaa    240
   ccagcaaagg tagcaacagt agaggttagc aatgttacct ttgtcaccgt cagtgacagg    300
   cacaagaacg aacttgttac actcgagata catagcttct gtaaaactga atcattcgcg    360
   gtttctgttt gctctgctga catctattgg tccattgttg gagactctgt tgctttttct    420
   gttttttaaa ttcttataga acccgctatt gctgtcgtgg tgagccatcc gccgcgaaac    480
   ggctgatgct cgactcattg tggctgcttt gcccattcgc tgcggttagt tgcagaaaaa 540
   aacagcggca caaatagctg ctcccccagg tgataatatc tctacaacag cattctagga 600
   tgtgcttgaa tgtcccgccc tcttcctttc caaatagttg ctcgcgcagt tgatgatatc 660
   gtaacagcag cgttctagtg agtatttgaa tacccagccc tgttgttttc aaagccacgc 720
   aacagcagca gttacagaag catataaagc ccatgcagct gatgctgctg cagctgaact 780
   ctctactggg aaaagcatgc atcatgcaaa atgctgtgca acctgcgggg caaagcatgc 840
   aaaaagctcc cctctaaggg gatgtgtgtg actctcaata ggcgtactgg ttttgctgcc 900
   agaagcttcc gctagtgtga ccaatacatt tagagtttgc cattaccact ggtgttaagg 960
   acctccgtaa agagcttttg gaaggcggca gttcgagcaa gcaggagctg gactgccttt 1020
   aacagccatt ggcaagtgaa accatcgaat cactgttact cacacagacg ggcatttgct 1080
   cgcacttttt tggttagggc ctcaagggaa tttggggcag cagtggatta gcacctccac 1140
   tgctcttttc gccaattctt ccgtcacccc gggtttacaa acgaacgagc tcgtagctgt 1200
   agtccccggc gtttgtactg tgccccgcag taagtgggac gcactaaggt cctcctactt 1260
   taacagttcg agttccttct tctcgcgctc ggctttccgc tctcgcatgt atcttgcacc 1320
   actgcaactg caacattcag tgacttagcc gtcagatggc atctgcagtg ctgttgtcat 1380
   taataaattt catcgttgct gtacatagcc tgtttatcac gcgactatat tgctaaacga 1440
   tgcaaagaca gaagtgccag cagcagcaga agcagcctaa acggcgcaaa ccctgtcggg 1500
   tggcagcccg ttaatttcga agcatagtca tgcgtgcttg cagcacttca gacactcaaa 1560
   aaaataatgc atatctccac acaaatgcag gcatattctc aacgtctcag taggttgcta 1620
   tgacctgaac tgtggggggt ggttgttata cagaaggcga cgtaaacgaa gtcctgcggg 1680
   gactgaacaa taatctacgg atgtatttag tcctaaaagc acaaacttga tggcagttgg 1740
   cagaagaaac atttaaagaa ttagttttaa ccaaaaggct acgagcgagg gaggaggcgc 1800
   ggggatgctg cagcacctcc tctaccaccc tgtgtacttc cgtacgttgc acaggtggcg 1860
   gtgaataacg tccgtcggtt ctacttgcat ccttaagctg ttattaaagc gtaattttgc 1920
   aaaaaattag gatagagaat aaaatatttg gaaaacaatg gcgctttatt tgctgaaaca 1980
   acaagcgagg ctgggggtcg gctgttcgcc cgggcgcggg ctagcaggct agctgcgcag 2040
   agactgcatg ttcccccact gtacgtgcag ggtgcgcgtg ttgttgttcg tgatggtatc 2100
   ggcgcgcgta tgtctcacat ccgttattta gaggctacaa cgagactgtc tgttgtgtct 2160
   gttccataga aaaccaaaat ggctcgtctt tcttttgttt ctcttctttc tctgtcactg 2220
   ctcttcgggc agcaagcagt cagagctcag aattacccgc gggagaaaat agtgcttctt 2280
   cttgcaatag tcagtcttgt taaaagtgat cagatttgca ttggttacca tgcaaacaac 2340
   tcgacagagc aggttgacac aataatggaa aagaacgtca ctgttacaca cgcccaagac 2400
   atactggaaa agacacacaa cgggaagctc tgcgatctag atggagtgaa gcctctaatt 2460
   ttaagagatt gtagtgtagc tggatggctc ctcgggaacc caatgtgtga cgaattcctc 2520
   aatgtgccgg aatggtctta catagtggag aagatcaatc cagccaatga cctctgttac 2580
   ccagggaatt tcaacgacta tgaagaactg aaacacctat tgagcagaat aaaccatttt 2640
   gagaaaattc agatcatccc caaaagttct tggtcagatc atgaagcctc gtcaggggtg 2700
   agctcagcat gtccatacca gggaaggtcc tcctttttta gaaatgtggt atggcttatc 2760
   aaaaagaaca atgcataccc aacaataaag agaagttaca ataataccaa ccaagaagat 2820
   cttttggtac tgtgggggat tcaccatcca aatgatgcgg cagagcagac aaggctctat 2880
   caaaacccaa ccacctatat ttccgttggg acatcaacac taaaccagag actggtacca 2940
   aaaatagcta ctagatccaa ggtaaacggg caaagtggaa ggatggagtt cttttggaca 3000
   attttaaaac cgaatgatgc aataaacttt gagagtaatg gaaatttcat tgctccagaa 3060
   aatgcataca aaattgtcag gaaaggggac tcaacaatta tgaaaagtga attggaatat 3120
   ggtaactgca acaccaagtg tcaaactcca ataggggcga taaactctag tatgccattc 3180
   cacaacatcc accctctcac catcggggaa tgccccaaat atgtgaaatc aaacagatta 3240
   atccttgcga ctgggctcag aaatagccct caaggagagg ggatccaagg aatggtgagc 3300
   aagggcgagg agctgttcac cggggtggtg cccatcctgg tcgagctgga cggcgacgta 3360
   aacggccaca agttcagcgt gtccggcgag ggcgagggcg atgccaccta cggcaagctg 3420
   accctgaagt tcatctgcac caccggcaag ctgcccgtgc cctggcccac cctcgtgacc 3480
   accttcggct acggcctgca gtgcttcgcc cgctaccccg accacatgaa gcagcacgac 3540
   ttcttcaagt ccgccatgcc cgaaggctac gtccaggagc gcaccatctt cttcaaggac 3600
   gacggcaact acaagacccg cgccgaggtg aagttcgagg gcgacaccct ggtgaaccgc 3660
   atcgagctga agggcatcga cttcaaggag gacggcaaca tcctggggca caagctggag 3720
   tacaactaca acagccacaa cgtctatatc atggccgaca agcagaagaa cggcatcaag 3780
   gtgaacttca agatccgcca caacatcgag gacggcagcg tgcagctcgc cgaccactac 3840
   cagcagaaca cccccatcgg cgacggcccc gtgctgctgc ccgacaacca ctacctgagc 3900
   taccagtccg ccctgagcaa agaccccaac gagaagcgcg atcacatggt cctgctggag 3960
   ttcgtgaccg ccgccgggat cactctcggc atggacgagc tgtacaagaa gctttaggcg 4020
   gccgcgtttg cagcagagta gttcatgact tgcgaaacgg cctctcgatt aataatacgc 4080
   tttgccgcaa cgtgaagtag gcgttattgt gtgctgcctg tcgctgagct cctgcatcga 4140
   gcggcaaggg gttcaaccga gcgcaaattc tgtggaaata gctggacaaa agcatctgcg 4200
   acgggtgggg gcaggcgcag ctagctgttg ctcccgacat cagtcatgga aatgcgtttc 4260
   aggctaagca agtagctgcc cgcctggtgg gaagatgagg ctaggaactg ctatgtttgc 4320
   ccctcacttg gcggtattgc agtgtagtac gtgcattgtg aacccagaga aatgtgctcc 4380
   gcgtgacgca gcgaggcaca ggggaacagc aagaggggcc gttattgctc agcgtgctgg 4440
   acccccttgt tccttcaaac cttacttatg agggcttacg catcgcacct gatgggtcgg 4500
   tgtagcgtga tcatttcact gttcagtagt aggtatggga ggagtgtagt tggcaggatg 4560
   taggagcttt gcaagcggtg gaacggttga gatgagtagt gaacagaggc tttgcccaat 4620
   gtcggatagt gagtgtttct cgagcacgaa acgtgcaagg caacaggtta ctgtaggttt   4680
   atgtagttgg caagagtggg ccttgcccag tattcggtag taagtgtcaa gcacgacacc   4740
   agattgcgaa gcaacaggta attgtaggtt tacgtatcgt acgtttttca tgggagtgtg   4800
   acggaaagtt gtgggtagca gggtccgggg gagtctgcgg caagcagttc aacagattga   4860
   tggaacagaa agattaagag gcggagtgtc cgctgttgct gtgggcagaa agagggcggc   4920
   gtagagaggc atttagtgga tgcttttgag gagttctggg aggtcatcag tagtggggaa   4980
   ggtctaccgc accgtctggt cggcctatta gttaatgccc acatgaggcg atcttttggt   5040
   ggtgcgtgac ggggtcagtc attggatttg gtatctgccg ctccatttgt agctaggaga   5100
   gcctctgatg ccgtgacatc ttgctaggtt cgcactgttt ccacaacgac tactttgttt   5160
   ggtcattttc accgtgaaag tatgttcacc ttggagagag aaaatttgag agagctctga   5220
   gttgttggaa cagcccaagt agctgactct cataggatac tactaaagcc aaacggcgcg   5280
   ttgtagcgct catctgcagg gattgatctt taaaaaaatg atcttttaag gttcatgcca   5340
   tacttattga gttcgctagg tgtcaacttg tcgaacacat tcttgaggtg ttgcgtatct   5400
   gggcgctccc aggggttcca tgtgaagcaa ttgttgttgt taacttgttt attgcagctt   5460
   ataatggtta caaataaagc aatagcatca caaatttcac aaataaagca tttttttcac   5520
   tgcattctag ttgtggtttg tccaaactca tcaatgtatc ttaaggcgta aattgtaagc   5580
   gttaatattt tgttaaaatt cgcgttaaat ttttgttaaa tcagctcatt ttttaaccaa   5640
   taggccgaaa tcggcaaaat cccttataaa tcaaaagaat agaccgagat agggttgagt   5700
   gttgttccag tttggaacaa gagtccacta ttaaagaacg tggactccaa cgtcaaaggg   5760
   cgaaaaaccg tctatcaggg cgatggccca ctacgtgaac catcacccta atcaagtttt   5820
   ttggggtcga ggtgccgtaa agcactaaat cggaacccta aagggagccc ccgatttaga   5880
   gcttgacggg gaaagccggc gaacgtggcg agaaaggaag ggaagaaagc gaaaggagcg   5940
   ggcgctaggg cgctggcaag tgtagcggtc acgctgcgcg taaccaccac acccgccgcg   6000
   cttaatgcgc cgctacaggg cgcgtcaggt ggcacttttc ggggaaatgt gcgcggaacc   6060
   cctatttgtt tatttttcta aatacattca aatatgtatc cgctcatgag acaataaccc   6120
   tgataaatgc ttcaataata ttgaaaaagg aagagtcctg aggcggaaag aaccagctgt   6180
   ggaatgtgtg tcagttaggg tgtggaaagt ccccaggctc cccagcaggc agaagtatgc   6240
   aaagcatgca tctcaattag tcagcaacca ggtgtggaaa gtccccaggc tccccagcag   6300
   gcagaagtat gcaaagcatg catctcaatt agtcagcaac catagtcccg cccctaactc   6360
   cgcccatccc gcccctaact ccgcccagtt ccgcccattc tccgccccat ggctgactaa   6420
   ttttttttat ttatgcagag gccgaggccg cctcggcctc tgagctattc cagaagtagt   6480
   gaggaggctt ttttggaggc ctaggctttt gcaaagatcg atcaagagac aggatgagga   6540
   tcgtttcgca tgattgaaca agatggattg cacgcaggtt ctccggccgc ttgggtggag   6600
   aggctattcg gctatgactg ggcacaacag acaatcggct gctctgatgc cgccgtgttc   6660
   cggctgtcag cgcaggggcg cccggttctt tttgtcaaga ccgacctgtc cggtgccctg   6720
   aatgaactgc aagacgaggc agcgcggcta tcgtggctgg ccacgacggg cgttccttgc   6780
   gcagctgtgc tcgacgttgt cactgaagcg ggaagggact ggctgctatt gggcgaagtg   6840
   ccggggcagg atctcctgtc atctcacctt gctcctgccg agaaagtatc catcatggct   6900
   gatgcaatgc ggcggctgca tacgcttgat ccggctacct gcccattcga ccaccaagcg   6960
   aaacatcgca tcgagcgagc acgtactcgg atggaagccg gtcttgtcga tcaggatgat   7020
   ctggacgaag agcatcaggg gctcgcgcca gccgaactgt tcgccaggct caaggcgagc   7080
   atgcccgacg gcgaggatct cgtcgtgacc catggcgatg cctgcttgcc gaatatcatg   7140
   gtggaaaatg gccgcttttc tggattcatc gactgtggcc ggctgggtgt ggcggaccgc   7200
   tatcaggaca tagcgttggc tacccgtgat attgctgaag agcttggcgg cgaatgggct   7260
   gaccgcttcc tcgtgcttta cggtatcgcc gctcccgatt cgcagcgcat cgccttctat   7320
   cgccttcttg acgagttctt ctgagcggga ctctggggtt cgaaatgacc gaccaagcga   7380
   cgcccaacct gccatcacga gatttcgatt ccaccgccgc cttctatgaa aggttgggct   7440
   tcggaatcgt tttccgggac gccggctgga tgatcctcca gcgcggggat ctcatgctgg   7500
   agttcttcgc ccaccctagg gggaggctaa ctgaaacacg gaaggagaca ataccggaag   7560
   gaacccgcgc tatgacggca ataaaaagac agaataaaac gcacggtgtt gggtcgtttg   7620
   ttcataaacg cggggttcgg tcccagggct ggcactctgt cgatacccca ccgagacccc   7680
   attggggcca atacgcccgc gtttcttcct tttccccacc ccacccccca agttcgggtg   7740
   aaggcccagg gctcgcagcc aacgtcgggg cggcaggccc tgccatagcc tcaggttact   7800
   catatatact ttagattgat ttaaaacttc atttttaatt taaaaggatc taggtgaaga   7860
   tcctttttga taatctcatg accaaaatcc cttaacgtga gttttcgttc cactgagcgt   7920
   cagaccccgt agaaaagatc aaaggatctt cttgagatcc tttttttctg cgcgtaatct   7980
   gctgcttgca aacaaaaaaa ccaccgctac cagcggtggt ttgtttgccg gatcaagagc   8040
   taccaactct ttttccgaag gtaactggct tcagcagagc gcagatacca aatactgtcc   8100
   ttctagtgta gccgtagtta ggccaccact tcaagaactc tgtagcaccg cctacatacc   8160
   tcgctctgct aatcctgtta ccagtggctg ctgccagtgg cgataagtcg tgtcttaccg   8220
   ggttggactc aagacgatag ttaccggata aggcgcagcg gtcgggctga acggggggtt   8280
   cgtgcacaca gcccagcttg gagcgaacga cctacaccga actgagatac ctacagcgtg   8340
   agctatgaga aagcgccacg cttcccgaag ggagaaaggc ggacaggtat ccggtaagcg   8400
   gcagggtcgg aacaggagag cgcacgaggg agcttccagg gggaaacgcc tggtatcttt   8460
   atagtcctgt cgggtttcgc cacctctgac ttgagcgtcg atttttgtga tgctcgtcag   8520
   gggggcggag cctatggaaa aacgccagca acgcggcctt tttacggttc ctggcctttt   8580
   gctggccttt tgctcacatg ttctttcctg cgttatcccc tgattctgtg gataaccgta   8640
   ttaccgccat gcat                                                      8654
<210> 3
   <211> 7694
   <212> DNA
   <213> artificial sequence
<400> 3
   tagttattaa tagtaatcaa ttacggggtc attagttcat agcccatata tggagttccg    60
   cgttacataa cttacggtaa atggcccgcc tggctgaccg cccaacgacc cccgcccatt   120
   gacgtcaata atgacgtatg ttcccatagt aacgccaata gggactttcc attgacgtca   180
   atgggtggag tatttacggt aaactgccca cttggcagta catcaagtgt atcatatgaa   240
   ccagcaaagg tagcaacagt agaggttagc aatgttacct ttgtcaccgt cagtgacagg   300
   cacaagaacg aacttgttac actcgagata catagcttct gtaaaactga atcattcgcg   360
   gtttctgttt gctctgctga catctattgg tccattgttg gagactctgt tgctttttct   420
   gttttttaaa ttcttataga acccgctatt gctgtcgtgg tgagccatcc gccgcgaaac   480
   ggctgatgct cgactcattg tggctgcttt gcccattcgc tgcggttagt tgcagaaaaa   540
   aacagcggca caaatagctg ctcccccagg tgataatatc tctacaacag cattctagga   600
   tgtgcttgaa tgtcccgccc tcttcctttc caaatagttg ctcgcgcagt tgatgatatc   660
   gtaacagcag cgttctagtg agtatttgaa tacccagccc tgttgttttc aaagccacgc   720
   aacagcagca gttacagaag catataaagc ccatgcagct gatgctgctg cagctgaact   780
   ctctactggg aaaagcatgc atcatgcaaa atgctgtgca acctgcgggg caaagcatgc   840
   aaaaagctcc cctctaaggg gatgtgtgtg actctcaata ggcgtactgg ttttgctgcc   900
   agaagcttcc gctagtgtga ccaatacatt tagagtttgc cattaccact ggtgttaagg   960
   acctccgtaa agagcttttg gaaggcggca gttcgagcaa gcaggagctg gactgccttt  1020
   aacagccatt ggcaagtgaa accatcgaat cactgttact cacacagacg ggcatttgct  1080
   cgcacttttt tggttagggc ctcaagggaa tttggggcag cagtggatta gcacctccac  1140
   tgctcttttc gccaattctt ccgtcacccc gggtttacaa acgaacgagc tcgtagctgt  1200
   agtccccggc gtttgtactg tgccccgcag taagtgggac gcactaaggt cctcctactt  1260
   taacagttcg agttccttct tctcgcgctc ggctttccgc tctcgcatgt atcttgcacc  1320
   actgcaactg caacattcag tgacttagcc gtcagatggc atctgcagtg ctgttgtcat  1380
   taataaattt catcgttgct gtacatagcc tgtttatcac gcgactatat tgctaaacga  1440
   tgcaaagaca gaagtgccag cagcagcaga agcagcctaa acggcgcaaa ccctgtcggg  1500
   tggcagcccg ttaatttcga agcatagtca tgcgtgcttg cagcacttca gacactcaaa  1560
   aaaataatgc atatctccac acaaatgcag gcatattctc aacgtctcag taggttgcta  1620
   tgacctgaac tgtggggggt ggttgttata cagaaggcga cgtaaacgaa gtcctgcggg  1680
   gactgaacaa taatctacgg atgtatttag tcctaaaagc acaaacttga tggcagttgg  1740
   cagaagaaac atttaaagaa ttagttttaa ccaaaaggct acgagcgagg gaggaggcgc  1800
   ggggatgctg cagcacctcc tctaccaccc tgtgtacttc cgtacgttgc acaggtggcg  1860
   gtgaataacg tccgtcggtt ctacttgcat ccttaagctg ttattaaagc gtaattttgc  1920
   aaaaaattag gatagagaat aaaatatttg gaaaacaatg gcgctttatt tgctgaaaca  1980
   acaagcgagg ctgggggtcg gctgttcgcc cgggcgcggg ctagcaggct agctgcgcag  2040
   agactgcatg ttcccccact gtacgtgcag ggtgcgcgtg ttgttgttcg tgatggtatc  2100
   ggcgcgcgta tgtctcacat ccgttattta gaggctacaa cgagactgtc tgttgtgtct  2160
   gttccataga aaaccaaaat ggctcgtctt tcttttgttt ctcttctttc tctgtcactg  2220
   ctcttcgggc agcaagcagt cagagctcag aattacccgc ggagtcttct aaccgaggtc  2280
   gaaacgccta ccagaaacga atgggagtgc agatgcagcg attcaagtga tatggtgagc  2340
   aagggcgagg agctgttcac cggggtggtg cccatcctgg tcgagctgga cggcgacgta  2400
   aacggccaca agttcagcgt gtccggcgag ggcgagggcg atgccaccta cggcaagctg  2460
   accctgaagt tcatctgcac caccggcaag ctgcccgtgc cctggcccac cctcgtgacc  2520
   accttcggct acggcctgca gtgcttcgcc cgctaccccg accacatgaa gcagcacgac  2580
   ttcttcaagt ccgccatgcc cgaaggctac gtccaggagc gcaccatctt cttcaaggac  2640
   gacggcaact acaagacccg cgccgaggtg aagttcgagg gcgacaccct ggtgaaccgc  2700
   atcgagctga agggcatcga cttcaaggag gacggcaaca tcctggggca caagctggag  2760
   tacaactaca acagccacaa cgtctatatc atggccgaca agcagaagaa cggcatcaag  2820
   gtgaacttca agatccgcca caacatcgag gacggcagcg tgcagctcgc cgaccactac  2880
   cagcagaaca cccccatcgg cgacggcccc gtgctgctgc ccgacaacca ctacctgagc  2940
   taccagtccg ccctgagcaa agaccccaac gagaagcgcg atcacatggt cctgctggag  3000
   ttcgtgaccg ccgccgggat cactctcggc atggacgagc tgtacaagaa gctttaggcg  3060
   gccgcgtttg cagcagagta gttcatgact tgcgaaacgg cctctcgatt aataatacgc  3120
   tttgccgcaa cgtgaagtag gcgttattgt gtgctgcctg tcgctgagct cctgcatcga  3180
   gcggcaaggg gttcaaccga gcgcaaattc tgtggaaata gctggacaaa agcatctgcg  3240
   acgggtgggg gcaggcgcag ctagctgttg ctcccgacat cagtcatgga aatgcgtttc  3300
   aggctaagca agtagctgcc cgcctggtgg gaagatgagg ctaggaactg ctatgtttgc  3360
   ccctcacttg gcggtattgc agtgtagtac gtgcattgtg aacccagaga aatgtgctcc  3420
   gcgtgacgca gcgaggcaca ggggaacagc aagaggggcc gttattgctc agcgtgctgg  3480
   acccccttgt tccttcaaac cttacttatg agggcttacg catcgcacct gatgggtcgg  3540
   tgtagcgtga tcatttcact gttcagtagt aggtatggga ggagtgtagt tggcaggatg  3600
   taggagcttt gcaagcggtg gaacggttga gatgagtagt gaacagaggc tttgcccaat  3660
   gtcggatagt gagtgtttct cgagcacgaa acgtgcaagg caacaggtta ctgtaggttt  3720
   atgtagttgg caagagtggg ccttgcccag tattcggtag taagtgtcaa gcacgacacc  3780
   agattgcgaa gcaacaggta attgtaggtt tacgtatcgt acgtttttca tgggagtgtg   3840
   acggaaagtt gtgggtagca gggtccgggg gagtctgcgg caagcagttc aacagattga   3900
   tggaacagaa agattaagag gcggagtgtc cgctgttgct gtgggcagaa agagggcggc   3960
   gtagagaggc atttagtgga tgcttttgag gagttctggg aggtcatcag tagtggggaa   4020
   ggtctaccgc accgtctggt cggcctatta gttaatgccc acatgaggcg atcttttggt   4080
   ggtgcgtgac ggggtcagtc attggatttg gtatctgccg ctccatttgt agctaggaga   4140
   gcctctgatg ccgtgacatc ttgctaggtt cgcactgttt ccacaacgac tactttgttt   4200
   ggtcattttc accgtgaaag tatgttcacc ttggagagag aaaatttgag agagctctga   4260
   gttgttggaa cagcccaagt agctgactct cataggatac tactaaagcc aaacggcgcg   4320
   ttgtagcgct catctgcagg gattgatctt taaaaaaatg atcttttaag gttcatgcca   4380
   tacttattga gttcgctagg tgtcaacttg tcgaacacat tcttgaggtg ttgcgtatct   4440
   gggcgctccc aggggttcca tgtgaagcaa ttgttgttgt taacttgttt attgcagctt   4500
   ataatggtta caaataaagc aatagcatca caaatttcac aaataaagca tttttttcac   4560
   tgcattctag ttgtggtttg tccaaactca tcaatgtatc ttaaggcgta aattgtaagc   4620
   gttaatattt tgttaaaatt cgcgttaaat ttttgttaaa tcagctcatt ttttaaccaa   4680
   taggccgaaa tcggcaaaat cccttataaa tcaaaagaat agaccgagat agggttgagt   4740
   gttgttccag tttggaacaa gagtccacta ttaaagaacg tggactccaa cgtcaaaggg   4800
   cgaaaaaccg tctatcaggg cgatggccca ctacgtgaac catcacccta atcaagtttt   4860
   ttggggtcga ggtgccgtaa agcactaaat cggaacccta aagggagccc ccgatttaga   4920
   gcttgacggg gaaagccggc gaacgtggcg agaaaggaag ggaagaaagc gaaaggagcg   4980
   ggcgctaggg cgctggcaag tgtagcggtc acgctgcgcg taaccaccac acccgccgcg   5040
   cttaatgcgc cgctacaggg cgcgtcaggt ggcacttttc ggggaaatgt gcgcggaacc   5100
   cctatttgtt tatttttcta aatacattca aatatgtatc cgctcatgag acaataaccc   5160
   tgataaatgc ttcaataata ttgaaaaagg aagagtcctg aggcggaaag aaccagctgt   5220
   ggaatgtgtg tcagttaggg tgtggaaagt ccccaggctc cccagcaggc agaagtatgc   5280
   aaagcatgca tctcaattag tcagcaacca ggtgtggaaa gtccccaggc tccccagcag   5340
   gcagaagtat gcaaagcatg catctcaatt agtcagcaac catagtcccg cccctaactc   5400
   cgcccatccc gcccctaact ccgcccagtt ccgcccattc tccgccccat ggctgactaa   5460
   ttttttttat ttatgcagag gccgaggccg cctcggcctc tgagctattc cagaagtagt   5520
   gaggaggctt ttttggaggc ctaggctttt gcaaagatcg atcaagagac aggatgagga   5580
   tcgtttcgca tgattgaaca agatggattg cacgcaggtt ctccggccgc ttgggtggag   5640
   aggctattcg gctatgactg ggcacaacag acaatcggct gctctgatgc cgccgtgttc   5700
   cggctgtcag cgcaggggcg cccggttctt tttgtcaaga ccgacctgtc cggtgccctg   5760
   aatgaactgc aagacgaggc agcgcggcta tcgtggctgg ccacgacggg cgttccttgc   5820
   gcagctgtgc tcgacgttgt cactgaagcg ggaagggact ggctgctatt gggcgaagtg   5880
   ccggggcagg atctcctgtc atctcacctt gctcctgccg agaaagtatc catcatggct   5940
   gatgcaatgc ggcggctgca tacgcttgat ccggctacct gcccattcga ccaccaagcg   6000
   aaacatcgca tcgagcgagc acgtactcgg atggaagccg gtcttgtcga tcaggatgat   6060
   ctggacgaag agcatcaggg gctcgcgcca gccgaactgt tcgccaggct caaggcgagc   6120
   atgcccgacg gcgaggatct cgtcgtgacc catggcgatg cctgcttgcc gaatatcatg   6180
   gtggaaaatg gccgcttttc tggattcatc gactgtggcc ggctgggtgt ggcggaccgc   6240
   tatcaggaca tagcgttggc tacccgtgat attgctgaag agcttggcgg cgaatgggct   6300
   gaccgcttcc tcgtgcttta cggtatcgcc gctcccgatt cgcagcgcat cgccttctat   6360
   cgccttcttg acgagttctt ctgagcggga ctctggggtt cgaaatgacc gaccaagcga   6420
   cgcccaacct gccatcacga gatttcgatt ccaccgccgc cttctatgaa aggttgggct   6480
   tcggaatcgt tttccgggac gccggctgga tgatcctcca gcgcggggat ctcatgctgg   6540
   agttcttcgc ccaccctagg gggaggctaa ctgaaacacg gaaggagaca ataccggaag   6600
   gaacccgcgc tatgacggca ataaaaagac agaataaaac gcacggtgtt gggtcgtttg   6660
   ttcataaacg cggggttcgg tcccagggct ggcactctgt cgatacccca ccgagacccc   6720
   attggggcca atacgcccgc gtttcttcct tttccccacc ccacccccca agttcgggtg   6780
   aaggcccagg gctcgcagcc aacgtcgggg cggcaggccc tgccatagcc tcaggttact   6840
   catatatact ttagattgat ttaaaacttc atttttaatt taaaaggatc taggtgaaga   6900
   tcctttttga taatctcatg accaaaatcc cttaacgtga gttttcgttc cactgagcgt   6960
   cagaccccgt agaaaagatc aaaggatctt cttgagatcc tttttttctg cgcgtaatct   7020
   gctgcttgca aacaaaaaaa ccaccgctac cagcggtggt ttgtttgccg gatcaagagc   7080
   taccaactct ttttccgaag gtaactggct tcagcagagc gcagatacca aatactgtcc   7140
   ttctagtgta gccgtagtta ggccaccact tcaagaactc tgtagcaccg cctacatacc   7200
   tcgctctgct aatcctgtta ccagtggctg ctgccagtgg cgataagtcg tgtcttaccg   7260
   ggttggactc aagacgatag ttaccggata aggcgcagcg gtcgggctga acggggggtt   7320
   cgtgcacaca gcccagcttg gagcgaacga cctacaccga actgagatac ctacagcgtg   7380
   agctatgaga aagcgccacg cttcccgaag ggagaaaggc ggacaggtat ccggtaagcg   7440
   gcagggtcgg aacaggagag cgcacgaggg agcttccagg gggaaacgcc tggtatcttt   7500
   atagtcctgt cgggtttcgc cacctctgac ttgagcgtcg atttttgtga tgctcgtcag   7560
   gggggcggag cctatggaaa aacgccagca acgcggcctt tttacggttc ctggcctttt   7620
   gctggccttt tgctcacatg ttctttcctg cgttatcccc tgattctgtg gataaccgta   7680
   ttaccgccat gcat                                                     7694
<210> 4
   <211> 9640
   <212> DNA
   <213> artificial sequence
<400> 4
   agatctaacc agcaaaggta gcaacagtag aggttagcaa tgttaccttt gtcaccgtca     60
   gtgacaggca caagaacgaa cttgttacac tcgagataca tagcttctgt aaaactgaat    120
   cattcgcggt ttctgtttgc tctgctgaca tctattggtc cattgttgga gactctgttg    180
   ctttttctgt tttttaaatt cttatagaac ccgctattgc tgtcgtggtg agccatccgc    240
   cgcgaaacgg ctgatgctcg actcattgtg gctgctttgc ccattcgctg cggttagttg    300
   cagaaaaaaa cagcggcaca aatagctgct cccccaggtg ataatatctc tacaacagca    360
   ttctaggatg tgcttgaatg tcccgccctc ttcctttcca aatagttgct cgcgcagttg    420
   atgatatcgt aacagcagcg ttctagtgag tatttgaata cccagccctg ttgttttcaa    480
   agccacgcaa cagcagcagt tacagaagca tataaagccc atgcagctga tgctgctgca    540
   gctgaactct ctactgggaa aagcatgcat catgcaaaat gctgtgcaac ctgcggggca    600
   aagcatgcaa aaagctcccc tctaagggga tgtgtgtgac tctcaatagg cgtactggtt    660
   ttgctgccag aagcttccgc tagtgtgacc aatacattta gagtttgcca ttaccactgg    720
   tgttaaggac ctccgtaaag agcttttgga aggcggcagt tcgagcaagc aggagctgga    780
   ctgcctttaa cagccattgg caagtgaaac catcgaatca ctgttactca cacagacggg    840
   catttgctcg cacttttttg gttagggcct caagggaatt tggggcagca gtggattagc    900
   acctccactg ctcttttcgc caattcttcc gtcaccccgg gtttacaaac gaacgagctc    960
   gtagctgtag tccccggcgt ttgtactgtg ccccgcagta agtgggacgc actaaggtcc   1020
   tcctacttta acagttcgag ttccttcttc tcgcgctcgg ctttccgctc tcgcatgtat   1080
   cttgcaccac tgcaactgca acattcagtg acttagccgt cagatggcat ctgcagtgct   1140
   gttgtcatta ataaatttca tcgttgctgt acatagcctg tttatcacgc gactatattg   1200
   ctaaacgatg caaagacaga agtgccagca gcagcagaag cagcctaaac ggcgcaaacc   1260
   ctgtcgggtg gcagcccgtt aatttcgaag catagtcatg cgtgcttgca gcacttcaga   1320
   cactcaaaaa aataatgcat atctccacac aaatgcaggc atattctcaa cgtctcagta   1380
   ggttgctatg acctgaactg tggggggtgg ttgttataca gaaggcgacg taaacgaagt   1440
   cctgcgggga ctgaacaata atctacggat gtatttagtc ctaaaagcac aaacttgatg   1500
   gcagttggca gaagaaacat ttaaagaatt agttttaacc aaaaggctac gagcgaggga   1560
   ggaggcgcgg ggatgctgca gcacctcctc taccaccctg tgtacttccg tacgttgcac   1620
   aggtggcggt gaataacgtc cgtcggttct acttgcatcc ttaagctgtt attaaagcgt   1680
   aattttgcaa aaaattagga tagagaataa aatatttgga aaacaatggc gctttatttg   1740
   ctgaaacaac aagcgaggct gggggtcggc tgttcgcccg ggcgcgggct agcaggctag   1800
   ctgcgcagag actgcatgtt cccccactgt acgtgcaggg tgcgcgtgtt gttgttcgtg   1860
   atggtatcgg cgcgcgtatg tctcacatcc gttatttaga ggctacaacg agactgtctg   1920
   ttgtgtctgt tccatagaaa accaaaatgg ctttaccatt gcgtgtttcg gccacggtgt   1980
   tcgtggtctt cgctgtcttt ggtgtagctc gcgccggtac cgtcgccacc atggtgagca   2040
   agggcgagga gctgttcacc ggggtggtgc ccatcctggt cgagctggac ggcgacgtaa   2100
   acggccacaa gttcagcgtg tccggcgagg gcgagggcga tgccacctac ggcaagctga   2160
   ccctgaagtt catctgcacc accggcaagc tgcccgtgcc ctggcccacc ctcgtgacca   2220
   ccttcggcta cggcctgcag tgcttcgccc gctaccccga ccacatgaag cagcacgact   2280
   tcttcaagtc cgccatgccc gaaggctacg tccaggagcg caccatcttc ttcaaggacg   2340
   acggcaacta caagacccgc gccgaggtga agttcgaggg cgacaccctg gtgaaccgca   2400
   tcgagctgaa gggcatcgac ttcaaggagg acggcaacat cctggggcac aagctggagt   2460
   acaactacaa cagccacaac gtctatatca tggccgacaa gcagaagaac ggcatcaagg   2520
   tgaacttcaa gatccgccac aacatcgagg acggcagcgt gcagctcgcc gaccactacc   2580
   agcagaacac ccccatcggc gacggccccg tgctgctgcc cgacaaccac tacctgagct   2640
   accagtccgc cctgagcaaa gaccccaacg agaagcgcga tcacatggtc ctgctggagt   2700
   tcgtgaccgc cgccgggatc actctcggca tggacgagct gtacaagaag cttagccaac   2760
   cggtcgccac catggagaaa atagtgcttc ttcttgcaat agtcagtctt gttaaaagtg   2820
   atcagatttg cattggttac catgcaaaca actcgacaga gcaggttgac acaataatgg   2880
   aaaagaacgt cactgttaca cacgcccaag acatactgga aaagacacac aacgggaagc   2940
   tctgcgatct agatggagtg aagcctctaa ttttaagaga ttgtagtgta gctggatggc   3000
   tcctcgggaa cccaatgtgt gacgaattcc tcaatgtgcc ggaatggtct tacatagtgg   3060
   agaagatcaa tccagccaat gacctctgtt acccagggaa tttcaacgac tatgaagaac   3120
   tgaaacacct attgagcaga ataaaccatt ttgagaaaat tcagatcatc cccaaaagtt   3180
   cttggtcaga tcatgaagcc tcgtcagggg tgagctcagc atgtccatac cagggaaggt   3240
   cctccttttt tagaaatgtg gtatggctta tcaaaaagaa caatgcatac ccaacaataa   3300
   agagaagtta caataatacc aaccaagaag atcttttggt actgtggggg attcaccatc   3360
   caaatgatgc ggcagagcag acaaggctct atcaaaaccc aaccacctat atttccgttg   3420
   ggacatcaac actaaaccag agattggtac caaaaatagc tactagatcc aaggtaaacg   3480
   ggcaaagtgg aaggatggag ttcttttgga caattttaaa accgaatgat gcaataaact   3540
   ttgagagtaa tggaaatttc attgctccag aaaatgcata caaaattgtc aggaaagggg   3600
   actcaacaat tatgaaaagt gaattggaat atggtaactg caacaccaag tgtcaaactc   3660
   caataggggc gataaactct agtatgccat tccacaacat ccaccctctc accatcgggg   3720
   aatgccccaa atatgtgaaa tcaaacagat taatccttgc gactgggctc agaaatagcc   3780
   ctcaaggaga gagaagaagg aaaaagagag gactatttgg agctatagca ggttttatag   3840
   agggaggatg gcagggaatg gtagatggtt ggtatgggta ccaccatagc aacgagcagg 3900
   ggagtgggta cgctgcagac aaagaatcca ctcaaaaggc aatagatgga gtcaccaata 3960
   aggtcaactc gatcattgac aaaatgaaca ctcagtttga ggccgttgga agggaattta 4020
   ataacttaga aaggagaata gaaaatttaa acaagaagat ggaagacgga ttcctagatg 4080
   tctggactta taatgctgaa cttctggttc tcatggaaaa tgagagaact ctagactttc 4140
   atgactcaaa tgtcaagaac ctttacgaca aggtccgact acagcttagg gataatgcaa 4200
   aggagcttgg taacggttgt ttcgagttct atcacagatg tgataatgaa tgtatggaaa 4260
   gtgtaagaaa cggaacgtat gactacccgc agtattcaga agaagcaaga ttaaaaagag 4320
   aggaaataag tggagtaaaa ttggaatcaa taggaactta ccaaatactg tcaatttatt 4380
   caacagtggc gagctcccta gcactggcaa tcatggtggc tggtctatct ttatggatgt 4440
   gctccaatgg atcgttacaa tgcagaattt gcatttaggc ggccgcggga attcgattgg 4500
   ccgcgtttgc agcagagtag ttcatgactt gcgaaacggc ctctcgatta ataatacgct 4560
   ttgccgcaac gtgaagtagg cgttattgtg tgctgcctgt cgctgagctc ctgcatcgag 4620
   cggcaagggg ttcaaccgag cgcaaattct gtggaaatag ctggacaaaa gcatctgcga 4680
   cgggtggggg caggcgcagc tagctgttgc tcccgacatc agtcatggaa atgcgtttca 4740
   ggctaagcaa gtagctgccc gcctggtggg aagatgaggc taggaactgc tatgtttgcc 4800
   cctcacttgg cggtattgca gtgtagtacg tgcattgtga acccagagaa atgtgctccg 4860
   cgtgacgcag cgaggcacag gggaacagca agaggggccg ttattgctca gcgtgctgga 4920
   cccccttgtt ccttcaaacc ttacttatga gggcttacgc atcgcacctg atgggtcggt 4980
   gtagcgtgat catttcactg ttcagtagta ggtatgggag gagtgtagtt ggcaggatgt 5040
   aggagctttg caagcggtgg aacggttgag atgagtagtg aacagaggct ttgcccaatg 5100
   tcggatagtg agtgtttctc gagcacgaaa cgtgcaaggc aacaggttac tgtaggttta 5160
   tgtagttggc aagagtgggc cttgcccagt attcggtagt aagtgtcaag cacgacacca 5220
   gattgcgaag caacaggtaa ttgtaggttt acgtatcgta cgtttttcat gggagtgtga 5280
   cggaaagttg tgggtagcag ggtccggggg agtctgcggc aagcagttca acagattgat 5340
   ggaacagaaa gattaagagg cggagtgtcc gctgttgctg tgggcagaaa gagggcggcg 5400
   tagagaggca tttagtggat gcttttgagg agttctggga ggtcatcagt agtggggaag 5460
   gtctaccgca ccgtctggtc ggcctattag ttaatgccca catgaggcga tcttttggtg 5520
   gtgcgtgacg gggtcagtca ttggatttgg tatctgccgc tccatttgta gctaggagag 5580
   cctctgatgc cgtgacatct tgctaggttc gcactgtttc cacaacgact actttgtttg 5640
   gtcattttca ccgtgaaagt atgttcacct tggagagaga aaatttgaga gagctctgag 5700
   ttgttggaac agcccaagta gctgactctc ataggatact actaaagcca aacggcgcgt 5760
   tgtagcgctc atctgcaggg attgatcttt aaaaaaatga tcttttaagg ttcatgccat 5820
   acttattgag ttcgctaggt gtcaacttgt cgaacacatt cttgaggtgt tgcgtatctg 5880
   ggcgctccca ggggttccat gtgaagcaat tgtccagcgt tgcgcggacg ctcggatgaa 5940
   accaggcagg aacggtaggt ttgcgacgat tgtacaagag cgcgtgtatt actcctacgc 6000
   atccacagaa atcagcattg gtaatcgtcg tggctgttgc aaggtctggt agcaaagctg 6060
   catatatttg cttgactgaa tttcacttac cctcgttaga gtgtatgtct tcgtagggta 6120
   tcggaccccc cagtatttca attaggcagc atgcaagccc ccaaatatca agcttctcat 6180
   ccacgtagtt gccttccacg aagcattcag gagccatgta gcggggagac cctccgttgt 6240
   cctccaattt aagctttccg tgctgctcta atgaccgtgt cttgccaaag tcgcagagcc 6300
   gaatgttgta ctgcacacat aagttgaaca tcacagagaa accaagggag aaattcactc 6360
   acagggctgt gcccaccctc attcatcctg gtagaatagc caaccaattg cacccacgtg 6420
   aagccaaaac gagcgaacat tcatgacagc aagttatgaa ttttggcgac ctgtgttggt 6480
   aaaaatgaaa aggaggctac gaagaatttg agcgtttggt gcgaatcctg cttacatctc 6540
   ggtcgcttaa ggcgtaaatt gtaagcgtta atattttgtt aaaattcgcg ttaaattttt 6600
   gttaaatcag ctcatttttt aaccaatagg ccgaaatcgg caaaatccct tataaatcaa 6660
   aagaatagac cgagataggg ttgagtgttg ttccagtttg gaacaagagt ccactattaa 6720
   agaacgtgga ctccaacgtc aaagggcgaa aaaccgtcta tcagggcgat ggcccactac 6780
   gtgaaccatc accctaatca agttttttgg ggtcgaggtg ccgtaaagca ctaaatcgga 6840
   accctaaagg gagcccccga tttagagctt gacggggaaa gccggcgaac gtggcgagaa 6900
   aggaagggaa gaaagcgaaa ggagcgggcg ctagggcgct ggcaagtgta gcggtcacgc 6960
   tgcgcgtaac caccacaccc gccgcgctta atgcgccgct acagggcgcg tcaggtggca 7020
   cttttcgggg aaatgtgcgc ggaaccccta tttgtttatt tttctaaata cattcaaata 7080
   tgtatccgct catgagacaa taaccctgat aaatgcttca ataatattga aaaaggaaga 7140
   gtcctgaggc ggaaagaacc agctgtggaa tgtgtgtcag ttagggtgtg gaaagtcccc 7200
   aggctcccca gcaggcagaa gtatgcaaag catgcatctc aattagtcag caaccaggtg 7260
   tggaaagtcc ccaggctccc cagcaggcag aagtatgcaa agcatgcatc tcaattagtc 7320
   agcaaccata gtcccgcccc taactccgcc catcccgccc ctaactccgc ccagttccgc 7380
   ccattctccg ccccatggct gactaatttt ttttatttat gcagaggccg aggccgcctc 7440
   ggcctctgag ctattccaga agtagtgagg aggctttttt ggaggcctag gcttttgcaa 7500
   agatcgatca agagacagga tgaggatcgt ttcgcatgat tgaacaagat ggattgcacg 7560
   caggttctcc ggccgcttgg gtggagaggc tattcggcta tgactgggca caacagacaa 7620
   tcggctgctc tgatgccgcc gtgttccggc tgtcagcgca ggggcgcccg gttctttttg 7680
   tcaagaccga cctgtccggt gccctgaatg aactgcaaga cgaggcagcg cggctatcgt 7740
   ggctggccac gacgggcgtt ccttgcgcag ctgtgctcga cgttgtcact gaagcgggaa 7800
   gggactggct gctattgggc gaagtgccgg ggcaggatct cctgtcatct caccttgctc 7860
   ctgccgagaa agtatccatc atggctgatg caatgcggcg gctgcatacg cttgatccgg 7920
   ctacctgccc attcgaccac caagcgaaac atcgcatcga gcgagcacgt actcggatgg 7980
   aagccggtct tgtcgatcag gatgatctgg acgaagagca tcaggggctc gcgccagccg    8040
   aactgttcgc caggctcaag gcgagcatgc ccgacggcga ggatctcgtc gtgacccatg    8100
   gcgatgcctg cttgccgaat atcatggtgg aaaatggccg cttttctgga ttcatcgact    8160
   gtggccggct gggtgtggcg gaccgctatc aggacatagc gttggctacc cgtgatattg    8220
   ctgaagagct tggcggcgaa tgggctgacc gcttcctcgt gctttacggt atcgccgctc    8280
   ccgattcgca gcgcatcgcc ttctatcgcc ttcttgacga gttcttctga gcgggactct    8340
   ggggttcgaa atgaccgacc aagcgacgcc caacctgcca tcacgagatt tcgattccac    8400
   cgccgccttc tatgaaaggt tgggcttcgg aatcgttttc cgggacgccg gctggatgat    8460
   cctccagcgc ggggatctca tgctggagtt cttcgcccac cctaggggga ggctaactga    8520
   aacacggaag gagacaatac cggaaggaac ccgcgctatg acggcaataa aaagacagaa    8580
   taaaacgcac ggtgttgggt cgtttgttca taaacgcggg gttcggtccc agggctggca    8640
   ctctgtcgat accccaccga gaccccattg gggccaatac gcccgcgttt cttccttttc    8700
   cccaccccac cccccaagtt cgggtgaagg cccagggctc gcagccaacg tcggggcggc    8760
   aggccctgcc atagcctcag gttactcata tatactttag attgatttaa aacttcattt    8820
   ttaatttaaa aggatctagg tgaagatcct ttttgataat ctcatgacca aaatccctta    8880
   acgtgagttt tcgttccact gagcgtcaga ccccgtagaa aagatcaaag gatcttcttg    8940
   agatcctttt tttctgcgcg taatctgctg cttgcaaaca aaaaaaccac cgctaccagc    9000
   ggtggtttgt ttgccggatc aagagctacc aactcttttt ccgaaggtaa ctggcttcag    9060
   cagagcgcag ataccaaata ctgtccttct agtgtagccg tagttaggcc accacttcaa    9120
   gaactctgta gcaccgccta catacctcgc tctgctaatc ctgttaccag tggctgctgc    9180
   cagtggcgat aagtcgtgtc ttaccgggtt ggactcaaga cgatagttac cggataaggc    9240
   gcagcggtcg ggctgaacgg ggggttcgtg cacacagccc agcttggagc gaacgaccta    9300
   caccgaactg agatacctac agcgtgagct atgagaaagc gccacgcttc ccgaagggag    9360
   aaaggcggac aggtatccgg taagcggcag ggtcggaaca ggagagcgca cgagggagct    9420
   tccaggggga aacgcctggt atctttatag tcctgtcggg tttcgccacc tctgacttga    9480
   gcgtcgattt ttgtgatgct cgtcaggggg gcggagccta tggaaaaacg ccagcaacgc    9540
   ggccttttta cggttcctgg ccttttgctg gccttttgct cacatgttct ttcctgcgtt    9600
   atcccctgat tctgtggata accgtattac cgccatgcat                          9640
<210> 5
   <211> 9343
   <212> DNA
   <213> artificial sequence
<400> 5
   agatctaacc agcaaaggta gcaacagtag aggttagcaa tgttaccttt gtcaccgtca      60
   gtgacaggca caagaacgaa cttgttacac tcgagataca tagcttctgt aaaactgaat     120
   cattcgcggt ttctgtttgc tctgctgaca tctattggtc cattgttgga gactctgttg     180
   ctttttctgt tttttaaatt cttatagaac ccgctattgc tgtcgtggtg agccatccgc     240
   cgcgaaacgg ctgatgctcg actcattgtg gctgctttgc ccattcgctg cggttagttg     300
   cagaaaaaaa cagcggcaca aatagctgct cccccaggtg ataatatctc tacaacagca     360
   ttctaggatg tgcttgaatg tcccgccctc ttcctttcca aatagttgct cgcgcagttg     420
   atgatatcgt aacagcagcg ttctagtgag tatttgaata cccagccctg ttgttttcaa     480
   agccacgcaa cagcagcagt tacagaagca tataaagccc atgcagctga tgctgctgca     540
   gctgaactct ctactgggaa aagcatgcat catgcaaaat gctgtgcaac ctgcggggca     600
   aagcatgcaa aaagctcccc tctaagggga tgtgtgtgac tctcaatagg cgtactggtt     660
   ttgctgccag aagcttccgc tagtgtgacc aatacattta gagtttgcca ttaccactgg     720
   tgttaaggac ctccgtaaag agcttttgga aggcggcagt tcgagcaagc aggagctgga     780
   ctgcctttaa cagccattgg caagtgaaac catcgaatca ctgttactca cacagacggg     840
   catttgctcg cacttttttg gttagggcct caagggaatt tggggcagca gtggattagc     900
   acctccactg ctcttttcgc caattcttcc gtcaccccgg gtttacaaac gaacgagctc     960
   gtagctgtag tccccggcgt ttgtactgtg ccccgcagta agtgggacgc actaaggtcc    1020
   tcctacttta acagttcgag ttccttcttc tcgcgctcgg ctttccgctc tcgcatgtat    1080
   cttgcaccac tgcaactgca acattcagtg acttagccgt cagatggcat ctgcagtgct    1140
   gttgtcatta ataaatttca tcgttgctgt acatagcctg tttatcacgc gactatattg    1200
   ctaaacgatg caaagacaga agtgccagca gcagcagaag cagcctaaac ggcgcaaacc    1260
   ctgtcgggtg gcagcccgtt aatttcgaag catagtcatg cgtgcttgca gcacttcaga    1320
   cactcaaaaa aataatgcat atctccacac aaatgcaggc atattctcaa cgtctcagta    1380
   ggttgctatg acctgaactg tggggggtgg ttgttataca gaaggcgacg taaacgaagt    1440
   cctgcgggga ctgaacaata atctacggat gtatttagtc ctaaaagcac aaacttgatg    1500
   gcagttggca gaagaaacat ttaaagaatt agttttaacc aaaaggctac gagcgaggga    1560
   ggaggcgcgg ggatgctgca gcacctcctc taccaccctg tgtacttccg tacgttgcac    1620
   aggtggcggt gaataacgtc cgtcggttct acttgcatcc ttaagctgtt attaaagcgt    1680
   aattttgcaa aaaattagga tagagaataa aatatttgga aaacaatggc gctttatttg    1740
   ctgaaacaac aagcgaggct gggggtcggc tgttcgcccg ggcgcgggct agcaggctag    1800
   ctgcgcagag actgcatgtt cccccactgt acgtgcaggg tgcgcgtgtt gttgttcgtg    1860
   atggtatcgg cgcgcgtatg tctcacatcc gttatttaga ggctacaacg agactgtctg    1920
   ttgtgtctgt tccatagaaa accaaaatgg ctttaccatt gcgtgtttcg gccacggtgt    1980
   tcgtggtctt cgctgtcttt ggtgtagctc gcgccggtac cgtcgccacc atggtgagca    2040
   agggcgagga gctgttcacc ggggtggtgc ccatcctggt cgagctggac ggcgacgtaa   2100
   acggccacaa gttcagcgtg tccggcgagg gcgagggcga tgccacctac ggcaagctga   2160
   ccctgaagtt catctgcacc accggcaagc tgcccgtgcc ctggcccacc ctcgtgacca   2220
   ccttcggcta cggcctgcag tgcttcgccc gctaccccga ccacatgaag cagcacgact   2280
   tcttcaagtc cgccatgccc gaaggctacg tccaggagcg caccatcttc ttcaaggacg   2340
   acggcaacta caagacccgc gccgaggtga agttcgaggg cgacaccctg gtgaaccgca   2400
   tcgagctgaa gggcatcgac ttcaaggagg acggcaacat cctggggcac aagctggagt   2460
   acaactacaa cagccacaac gtctatatca tggccgacaa gcagaagaac ggcatcaagg   2520
   tgaacttcaa gatccgccac aacatcgagg acggcagcgt gcagctcgcc gaccactacc   2580
   agcagaacac ccccatcggc gacggccccg tgctgctgcc cgacaaccac tacctgagct   2640
   accagtccgc cctgagcaaa gaccccaacg agaagcgcga tcacatggtc ctgctggagt   2700
   tcgtgaccgc cgccgggatc actctcggca tggacgagct gtacaagaag cttagccaac   2760
   cggtcgccac catgaatcca aatcagaaga taataaccat tggatcaatc tgtatgataa   2820
   ttggaatagt tagcttgatg ttacaaattg ggaacataat ctcaatatgg gttagtcatt   2880
   caattcagac agggaatcaa caccaagctg aaccatgcaa tcaaagcatt attacttatg   2940
   aaaacaacac ctgggtaaat cagacatatg tcaacatcag caataccaat tttcttactg   3000
   agaaagctgt ggcttcagta acattagcgg gcaattcatc tctttgccct attagtggat   3060
   gggctgtata cagtaaagac aacggtataa gaatcggttc caagggggat gtgtttgtta   3120
   taagagagcc gttcatctca tgctcccact tggaatgcag aactttcttt ttgactcagg   3180
   gagccttgct gaatgacaag cattctaatg ggaccgtcaa agacagaagc ccttacagaa   3240
   cattgatgag ttgtcctgtg ggtgaggctc cttccccata taactcgagg tttgagtctg   3300
   ttgcttggtc ggcaagtgct tgtcatgatg gcactagttg gttgacaatt ggaatttctg   3360
   gcccagacaa tggggctgtg gctgtattga aatacaatgg cataataaca gacacaatca   3420
   agagttggag gaacaacata ctgagaactc aagagtctga atgtgcatgt gtaaatggct   3480
   cttgctttac tgtaatgact gatggaccaa gtaatgggca ggcttcatat aagatcttca   3540
   aaatagaaaa agggaaagta gttaaatcag tcgaattgaa tgcccctaat tatcactatg   3600
   aggagtgctc ctgttatcct gatgctggcg aaatcacatg tgtgtgcagg gataactggc   3660
   atggctcaaa tcggccatgg gtatctttca atcaaaattt ggagtatcaa ataggatata   3720
   tatgcagtgg agttttcgga gacaatccac gccccaatga tgggacaggc agttgtggtc   3780
   cggtgtcccc taacggggca tatggaataa aagggttttc atttaaatac ggcaatggtg   3840
   tttggatcgg gagaaccaaa agcactaatt ccaggagcgg ctttgaaatg atttgggatc   3900
   caaatgggtg gactggaacg gacagtaatt tttcggtgaa gcaagatatc gtagctataa   3960
   ctgattggtc aggatatagc gggagttttg tccagcatcc agaactgaca ggattagatt   4020
   gcataagacc ttgtttctgg gttgagctaa tcagagggcg gcccaaagag agcacaattt   4080
   ggactagtgg gagcagcata tccttttgtg gtgtaaatag tgacactgtg ggttggtctt   4140
   ggccagacgg tgctgagttg ccattcacca ttgacaagta ggcggccgcg ggaattcgat   4200
   tggccgcgtt tgcagcagag tagttcatga cttgcgaaac ggcctctcga ttaataatac   4260
   gctttgccgc aacgtgaagt aggcgttatt gtgtgctgcc tgtcgctgag ctcctgcatc   4320
   gagcggcaag gggttcaacc gagcgcaaat tctgtggaaa tagctggaca aaagcatctg   4380
   cgacgggtgg gggcaggcgc agctagctgt tgctcccgac atcagtcatg gaaatgcgtt   4440
   tcaggctaag caagtagctg cccgcctggt gggaagatga ggctaggaac tgctatgttt   4500
   gcccctcact tggcggtatt gcagtgtagt acgtgcattg tgaacccaga gaaatgtgct   4560
   ccgcgtgacg cagcgaggca caggggaaca gcaagagggg ccgttattgc tcagcgtgct   4620
   ggaccccctt gttccttcaa accttactta tgagggctta cgcatcgcac ctgatgggtc   4680
   ggtgtagcgt gatcatttca ctgttcagta gtaggtatgg gaggagtgta gttggcagga   4740
   tgtaggagct ttgcaagcgg tggaacggtt gagatgagta gtgaacagag gctttgccca   4800
   atgtcggata gtgagtgttt ctcgagcacg aaacgtgcaa ggcaacaggt tactgtaggt   4860
   ttatgtagtt ggcaagagtg ggccttgccc agtattcggt agtaagtgtc aagcacgaca   4920
   ccagattgcg aagcaacagg taattgtagg tttacgtatc gtacgttttt catgggagtg   4980
   tgacggaaag ttgtgggtag cagggtccgg gggagtctgc ggcaagcagt tcaacagatt   5040
   gatggaacag aaagattaag aggcggagtg tccgctgttg ctgtgggcag aaagagggcg   5100
   gcgtagagag gcatttagtg gatgcttttg aggagttctg ggaggtcatc agtagtgggg   5160
   aaggtctacc gcaccgtctg gtcggcctat tagttaatgc ccacatgagg cgatcttttg   5220
   gtggtgcgtg acggggtcag tcattggatt tggtatctgc cgctccattt gtagctagga   5280
   gagcctctga tgccgtgaca tcttgctagg ttcgcactgt ttccacaacg actactttgt   5340
   ttggtcattt tcaccgtgaa agtatgttca ccttggagag agaaaatttg agagagctct   5400
   gagttgttgg aacagcccaa gtagctgact ctcataggat actactaaag ccaaacggcg   5460
   cgttgtagcg ctcatctgca gggattgatc tttaaaaaaa tgatctttta aggttcatgc   5520
   catacttatt gagttcgcta ggtgtcaact tgtcgaacac attcttgagg tgttgcgtat   5580
   ctgggcgctc ccaggggttc catgtgaagc aattgtccag cgttgcgcgg acgctcggat   5640
   gaaaccaggc aggaacggta ggtttgcgac gattgtacaa gagcgcgtgt attactccta   5700
   cgcatccaca gaaatcagca ttggtaatcg tcgtggctgt tgcaaggtct ggtagcaaag   5760
   ctgcatatat ttgcttgact gaatttcact taccctcgtt agagtgtatg tcttcgtagg   5820
   gtatcggacc ccccagtatt tcaattaggc agcatgcaag cccccaaata tcaagcttct   5880
   catccacgta gttgccttcc acgaagcatt caggagccat gtagcgggga gaccctccgt   5940
   tgtcctccaa tttaagcttt ccgtgctgct ctaatgaccg tgtcttgcca aagtcgcaga   6000
   gccgaatgtt gtactgcaca cataagttga acatcacaga gaaaccaagg gagaaattca   6060
   ctcacagggc tgtgcccacc ctcattcatc ctggtagaat agccaaccaa ttgcacccac   6120
   gtgaagccaa aacgagcgaa cattcatgac agcaagttat gaattttggc gacctgtgtt   6180
   ggtaaaaatg aaaaggaggc tacgaagaat ttgagcgttt ggtgcgaatc ctgcttacat 6240
   ctcggtcgct taaggcgtaa attgtaagcg ttaatatttt gttaaaattc gcgttaaatt 6300
   tttgttaaat cagctcattt tttaaccaat aggccgaaat cggcaaaatc ccttataaat 6360
   caaaagaata gaccgagata gggttgagtg ttgttccagt ttggaacaag agtccactat 6420
   taaagaacgt ggactccaac gtcaaagggc gaaaaaccgt ctatcagggc gatggcccac 6480
   tacgtgaacc atcaccctaa tcaagttttt tggggtcgag gtgccgtaaa gcactaaatc 6540
   ggaaccctaa agggagcccc cgatttagag cttgacgggg aaagccggcg aacgtggcga 6600
   gaaaggaagg gaagaaagcg aaaggagcgg gcgctagggc gctggcaagt gtagcggtca 6660
   cgctgcgcgt aaccaccaca cccgccgcgc ttaatgcgcc gctacagggc gcgtcaggtg 6720
   gcacttttcg gggaaatgtg cgcggaaccc ctatttgttt atttttctaa atacattcaa 6780
   atatgtatcc gctcatgaga caataaccct gataaatgct tcaataatat tgaaaaagga 6840
   agagtcctga ggcggaaaga accagctgtg gaatgtgtgt cagttagggt gtggaaagtc 6900
   cccaggctcc ccagcaggca gaagtatgca aagcatgcat ctcaattagt cagcaaccag 6960
   gtgtggaaag tccccaggct ccccagcagg cagaagtatg caaagcatgc atctcaatta 7020
   gtcagcaacc atagtcccgc ccctaactcc gcccatcccg cccctaactc cgcccagttc 7080
   cgcccattct ccgccccatg gctgactaat tttttttatt tatgcagagg ccgaggccgc 7140
   ctcggcctct gagctattcc agaagtagtg aggaggcttt tttggaggcc taggcttttg 7200
   caaagatcga tcaagagaca ggatgaggat cgtttcgcat gattgaacaa gatggattgc 7260
   acgcaggttc tccggccgct tgggtggaga ggctattcgg ctatgactgg gcacaacaga 7320
   caatcggctg ctctgatgcc gccgtgttcc ggctgtcagc gcaggggcgc ccggttcttt 7380
   ttgtcaagac cgacctgtcc ggtgccctga atgaactgca agacgaggca gcgcggctat 7440
   cgtggctggc cacgacgggc gttccttgcg cagctgtgct cgacgttgtc actgaagcgg 7500
   gaagggactg gctgctattg ggcgaagtgc cggggcagga tctcctgtca tctcaccttg 7560
   ctcctgccga gaaagtatcc atcatggctg atgcaatgcg gcggctgcat acgcttgatc 7620
   cggctacctg cccattcgac caccaagcga aacatcgcat cgagcgagca cgtactcgga 7680
   tggaagccgg tcttgtcgat caggatgatc tggacgaaga gcatcagggg ctcgcgccag 7740
   ccgaactgtt cgccaggctc aaggcgagca tgcccgacgg cgaggatctc gtcgtgaccc 7800
   atggcgatgc ctgcttgccg aatatcatgg tggaaaatgg ccgcttttct ggattcatcg 7860
   actgtggccg gctgggtgtg gcggaccgct atcaggacat agcgttggct acccgtgata 7920
   ttgctgaaga gcttggcggc gaatgggctg accgcttcct cgtgctttac ggtatcgccg 7980
   ctcccgattc gcagcgcatc gccttctatc gccttcttga cgagttcttc tgagcgggac 8040
   tctggggttc gaaatgaccg accaagcgac gcccaacctg ccatcacgag atttcgattc 8100
   caccgccgcc ttctatgaaa ggttgggctt cggaatcgtt ttccgggacg ccggctggat 8160
   gatcctccag cgcggggatc tcatgctgga gttcttcgcc caccctaggg ggaggctaac 8220
   tgaaacacgg aaggagacaa taccggaagg aacccgcgct atgacggcaa taaaaagaca 8280
   gaataaaacg cacggtgttg ggtcgtttgt tcataaacgc ggggttcggt cccagggctg 8340
   gcactctgtc gataccccac cgagacccca ttggggccaa tacgcccgcg tttcttcctt 8400
   ttccccaccc caccccccaa gttcgggtga aggcccaggg ctcgcagcca acgtcggggc 8460
   ggcaggccct gccatagcct caggttactc atatatactt tagattgatt taaaacttca 8520
   tttttaattt aaaaggatct aggtgaagat cctttttgat aatctcatga ccaaaatccc 8580
   ttaacgtgag ttttcgttcc actgagcgtc agaccccgta gaaaagatca aaggatcttc 8640
   ttgagatcct ttttttctgc gcgtaatctg ctgcttgcaa acaaaaaaac caccgctacc 8700
   agcggtggtt tgtttgccgg atcaagagct accaactctt tttccgaagg taactggctt 8760
   cagcagagcg cagataccaa atactgtcct tctagtgtag ccgtagttag gccaccactt 8820
   caagaactct gtagcaccgc ctacatacct cgctctgcta atcctgttac cagtggctgc 8880
   tgccagtggc gataagtcgt gtcttaccgg gttggactca agacgatagt taccggataa 8940
   ggcgcagcgg tcgggctgaa cggggggttc gtgcacacag cccagcttgg agcgaacgac 9000
   ctacaccgaa ctgagatacc tacagcgtga gctatgagaa agcgccacgc ttcccgaagg 9060
   gagaaaggcg gacaggtatc cggtaagcgg cagggtcgga acaggagagc gcacgaggga 9120
   gcttccaggg ggaaacgcct ggtatcttta tagtcctgtc gggtttcgcc acctctgact 9180
   tgagcgtcga tttttgtgat gctcgtcagg ggggcggagc ctatggaaaa acgccagcaa 9240
   cgcggccttt ttacggttcc tggccttttg ctggcctttt gctcacatgt tctttcctgc 9300
   gttatcccct gattctgtgg ataaccgtat taccgccatg cat                  9343
<210> 6
   <211> 9430
   <212> DNA
   <213> artificial sequence
<400> 6
   agatctaacc agcaaaggta gcaacagtag aggttagcaa tgttaccttt gtcaccgtca     60
   gtgacaggca caagaacgaa cttgttacac tcgagataca tagcttctgt aaaactgaat    120
   cattcgcggt ttctgtttgc tctgctgaca tctattggtc cattgttgga gactctgttg    180
   ctttttctgt tttttaaatt cttatagaac ccgctattgc tgtcgtggtg agccatccgc    240
   cgcgaaacgg ctgatgctcg actcattgtg gctgctttgc ccattcgctg cggttagttg    300
   cagaaaaaaa cagcggcaca aatagctgct cccccaggtg ataatatctc tacaacagca    360
   ttctaggatg tgcttgaatg tcccgccctc ttcctttcca aatagttgct cgcgcagttg    420
   atgatatcgt aacagcagcg ttctagtgag tatttgaata cccagccctg ttgttttcaa    480
   agccacgcaa cagcagcagt tacagaagca tataaagccc atgcagctga tgctgctgca    540
   gctgaactct ctactgggaa aagcatgcat catgcaaaat gctgtgcaac ctgcggggca 600
   aagcatgcaa aaagctcccc tctaagggga tgtgtgtgac tctcaatagg cgtactggtt 660
   ttgctgccag aagcttccgc tagtgtgacc aatacattta gagtttgcca ttaccactgg 720
   tgttaaggac ctccgtaaag agcttttgga aggcggcagt tcgagcaagc aggagctgga 780
   ctgcctttaa cagccattgg caagtgaaac catcgaatca ctgttactca cacagacggg 840
   catttgctcg cacttttttg gttagggcct caagggaatt tggggcagca gtggattagc 900
   acctccactg ctcttttcgc caattcttcc gtcaccccgg gtttacaaac gaacgagctc 960
   gtagctgtag tccccggcgt ttgtactgtg ccccgcagta agtgggacgc actaaggtcc 1020
   tcctacttta acagttcgag ttccttcttc tcgcgctcgg ctttccgctc tcgcatgtat 1080
   cttgcaccac tgcaactgca acattcagtg acttagccgt cagatggcat ctgcagtgct 1140
   gttgtcatta ataaatttca tcgttgctgt acatagcctg tttatcacgc gactatattg 1200
   ctaaacgatg caaagacaga agtgccagca gcagcagaag cagcctaaac ggcgcaaacc 1260
   ctgtcgggtg gcagcccgtt aatttcgaag catagtcatg cgtgcttgca gcacttcaga 1320
   cactcaaaaa aataatgcat atctccacac aaatgcaggc atattctcaa cgtctcagta 1380
   ggttgctatg acctgaactg tggggggtgg ttgttataca gaaggcgacg taaacgaagt 1440
   cctgcgggga ctgaacaata atctacggat gtatttagtc ctaaaagcac aaacttgatg 1500
   gcagttggca gaagaaacat ttaaagaatt agttttaacc aaaaggctac gagcgaggga 1560
   ggaggcgcgg ggatgctgca gcacctcctc taccaccctg tgtacttccg tacgttgcac 1620
   aggtggcggt gaataacgtc cgtcggttct acttgcatcc ttaagctgtt attaaagcgt 1680
   aattttgcaa aaaattagga tagagaataa aatatttgga aaacaatggc gctttatttg 1740
   ctgaaacaac aagcgaggct gggggtcggc tgttcgcccg ggcgcgggct agcaggctag 1800
   ctgcgcagag actgcatgtt cccccactgt acgtgcaggg tgcgcgtgtt gttgttcgtg 1860
   atggtatcgg cgcgcgtatg tctcacatcc gttatttaga ggctacaacg agactgtctg 1920
   ttgtgtctgt tccatagaaa accaaaatgg ctttaccatt gcgtgtttcg gccacggtgt 1980
   tcgtggtctt cgctgtcttt ggtgtagctc gcgccggtac cgtcgccacc atggtgagca 2040
   agggcgagga gctgttcacc ggggtggtgc ccatcctggt cgagctggac ggcgacgtaa 2100
   acggccacaa gttcagcgtg tccggcgagg gcgagggcga tgccacctac ggcaagctga 2160
   ccctgaagtt catctgcacc accggcaagc tgcccgtgcc ctggcccacc ctcgtgacca 2220
   ccttcggcta cggcctgcag tgcttcgccc gctaccccga ccacatgaag cagcacgact 2280
   tcttcaagtc cgccatgccc gaaggctacg tccaggagcg caccatcttc ttcaaggacg 2340
   acggcaacta caagacccgc gccgaggtga agttcgaggg cgacaccctg gtgaaccgca 2400
   tcgagctgaa gggcatcgac ttcaaggagg acggcaacat cctggggcac aagctggagt 2460
   acaactacaa cagccacaac gtctatatca tggccgacaa gcagaagaac ggcatcaagg 2520
   tgaacttcaa gatccgccac aacatcgagg acggcagcgt gcagctcgcc gaccactacc 2580
   agcagaacac ccccatcggc gacggccccg tgctgctgcc cgacaaccac tacctgagct 2640
   accagtccgc cctgagcaaa gaccccaacg agaagcgcga tcacatggtc ctgctggagt 2700
   tcgtgaccgc cgccgggatc actctcggca tggacgagct gtacaagaag cttagccaac 2760
   cggtcgccac catggcgtct caaggcacca aacgatctta tgaacagatg gaaactggtg 2820
   gagaacgcca gaatgctact gagatcagag catctgttgg aagaatggtc ggtggaattg 2880
   ggaggtttta tatacagatg tgcactgaac tcaaactcag tgactatgaa gggaggctga 2940
   ttcagaacag cataacaata gagagaatgg ttctctctgc atttgatgaa aggaggaaca 3000
   aatacctgga agaacatccc agtgcgggga aagacccaaa gaaaactgga ggtccaatct 3060
   accgaaggag agacgggaag tgggtgagag agctgattct gtatgataaa gaggagatca 3120
   ggaggatttg gcgccaagcg aacaatggag aagatgcaac tgctggtctc actcatctga 3180
   tgatctggca ttccaatcta aatgatgcca cataccagag gacaagagct ctcgtgcgta 3240
   ctgggatgga ccccagaatg tgttctctaa tgcaaggatc aactctcccg aggagatctg 3300
   gagctgcagg tgcagcagta aagggagtcg gaacgatggt gatggaacta attcggatga 3360
   taaagcgagg gattaatgat cggaatttct ggagaggtga aaatgggcga agaacgagga 3420
   ttgcatatga gagaatgtgc aacatcctca aagggaaatt ccaaacagca gcacaaagag 3480
   caatgatgga ccaggtacgg gaaagcagga atcctgggaa tgccgaaatt gaagatctca 3540
   tcttcctggc acggtctgca ctcatcctga gaggatcagt ggctcacaag tcctgcttgc 3600
   ctgcttgtgt gtacgggctt gctgtagcca gtggatatga ctttgagaga gaagggtatt 3660
   ctctggtcgg gattgatccc tttcgtctgc tgcaaaacag ccaagtcttc agtctaatta 3720
   gaccaaatga gaacccagca cataaaagtc aactggtatg gatggcatgc cattctgcag 3780
   catttgaaga tctgagggtc tcaagtttca tcagagggac aagagtagtt ccaaggggac 3840
   aactatctac tagaggagtt caaattgctt caaatgagaa catggataca atggactcca 3900
   acactctaga actgagaagc agatattggg ctataagaac caggagtgga ggaaacacca 3960
   atcagcagag agcatctgca ggacaaatca gtgtacagcc tactttttcg gtacagagaa 4020
   atcttccctt cgaaagagcg accattatgg cggcatttac agggaataca gagggcagaa 4080
   catctgacat gaggactgaa ataataagaa taatggaaag cgcccgacca gaagatgtgt 4140
   ctttccaggg gcggggagtc ttcgagctct cggacgaaaa ggcaacgaac ccgatcgtgc 4200
   cttcctttga catgagtaat gaaggatctt acttcttcgg agacaatgca gaggagtttg 4260
   agaattaagc ggccgcggga attcgattgg ccgcgtttgc agcagagtag ttcatgactt 4320
   gcgaaacggc ctctcgatta ataatacgct ttgccgcaac gtgaagtagg cgttattgtg 4380
   tgctgcctgt cgctgagctc ctgcatcgag cggcaagggg ttcaaccgag cgcaaattct 4440
   gtggaaatag ctggacaaaa gcatctgcga cgggtggggg caggcgcagc tagctgttgc 4500
   tcccgacatc agtcatggaa atgcgtttca ggctaagcaa gtagctgccc gcctggtggg 4560
   aagatgaggc taggaactgc tatgtttgcc cctcacttgg cggtattgca gtgtagtacg 4620
   tgcattgtga acccagagaa atgtgctccg cgtgacgcag cgaggcacag gggaacagca 4680
   agaggggccg ttattgctca gcgtgctgga cccccttgtt ccttcaaacc ttacttatga 4740
   gggcttacgc atcgcacctg atgggtcggt gtagcgtgat catttcactg ttcagtagta 4800
   ggtatgggag gagtgtagtt ggcaggatgt aggagctttg caagcggtgg aacggttgag 4860
   atgagtagtg aacagaggct ttgcccaatg tcggatagtg agtgtttctc gagcacgaaa 4920
   cgtgcaaggc aacaggttac tgtaggttta tgtagttggc aagagtgggc cttgcccagt 4980
   attcggtagt aagtgtcaag cacgacacca gattgcgaag caacaggtaa ttgtaggttt 5040
   acgtatcgta cgtttttcat gggagtgtga cggaaagttg tgggtagcag ggtccggggg 5100
   agtctgcggc aagcagttca acagattgat ggaacagaaa gattaagagg cggagtgtcc 5160
   gctgttgctg tgggcagaaa gagggcggcg tagagaggca tttagtggat gcttttgagg 5220
   agttctggga ggtcatcagt agtggggaag gtctaccgca ccgtctggtc ggcctattag 5280
   ttaatgccca catgaggcga tcttttggtg gtgcgtgacg gggtcagtca ttggatttgg 5340
   tatctgccgc tccatttgta gctaggagag cctctgatgc cgtgacatct tgctaggttc 5400
   gcactgtttc cacaacgact actttgtttg gtcattttca ccgtgaaagt atgttcacct 5460
   tggagagaga aaatttgaga gagctctgag ttgttggaac agcccaagta gctgactctc 5520
   ataggatact actaaagcca aacggcgcgt tgtagcgctc atctgcaggg attgatcttt 5580
   aaaaaaatga tcttttaagg ttcatgccat acttattgag ttcgctaggt gtcaacttgt 5640
   cgaacacatt cttgaggtgt tgcgtatctg ggcgctccca ggggttccat gtgaagcaat 5700
   tgtccagcgt tgcgcggacg ctcggatgaa accaggcagg aacggtaggt ttgcgacgat 5760
   tgtacaagag cgcgtgtatt actcctacgc atccacagaa atcagcattg gtaatcgtcg 5820
   tggctgttgc aaggtctggt agcaaagctg catatatttg cttgactgaa tttcacttac 5880
   cctcgttaga gtgtatgtct tcgtagggta tcggaccccc cagtatttca attaggcagc 5940
   atgcaagccc ccaaatatca agcttctcat ccacgtagtt gccttccacg aagcattcag 6000
   gagccatgta gcggggagac cctccgttgt cctccaattt aagctttccg tgctgctcta 6060
   atgaccgtgt cttgccaaag tcgcagagcc gaatgttgta ctgcacacat aagttgaaca 6120
   tcacagagaa accaagggag aaattcactc acagggctgt gcccaccctc attcatcctg 6180
   gtagaatagc caaccaattg cacccacgtg aagccaaaac gagcgaacat tcatgacagc 6240
   aagttatgaa ttttggcgac ctgtgttggt aaaaatgaaa aggaggctac gaagaatttg 6300
   agcgtttggt gcgaatcctg cttacatctc ggtcgcttaa ggcgtaaatt gtaagcgtta 6360
   atattttgtt aaaattcgcg ttaaattttt gttaaatcag ctcatttttt aaccaatagg 6420
   ccgaaatcgg caaaatccct tataaatcaa aagaatagac cgagataggg ttgagtgttg 6480
   ttccagtttg gaacaagagt ccactattaa agaacgtgga ctccaacgtc aaagggcgaa 6540
   aaaccgtcta tcagggcgat ggcccactac gtgaaccatc accctaatca agttttttgg 6600
   ggtcgaggtg ccgtaaagca ctaaatcgga accctaaagg gagcccccga tttagagctt 6660
   gacggggaaa gccggcgaac gtggcgagaa aggaagggaa gaaagcgaaa ggagcgggcg 6720
   ctagggcgct ggcaagtgta gcggtcacgc tgcgcgtaac caccacaccc gccgcgctta 6780
   atgcgccgct acagggcgcg tcaggtggca cttttcgggg aaatgtgcgc ggaaccccta 6840
   tttgtttatt tttctaaata cattcaaata tgtatccgct catgagacaa taaccctgat 6900
   aaatgcttca ataatattga aaaaggaaga gtcctgaggc ggaaagaacc agctgtggaa 6960
   tgtgtgtcag ttagggtgtg gaaagtcccc aggctcccca gcaggcagaa gtatgcaaag 7020
   catgcatctc aattagtcag caaccaggtg tggaaagtcc ccaggctccc cagcaggcag 7080
   aagtatgcaa agcatgcatc tcaattagtc agcaaccata gtcccgcccc taactccgcc 7140
   catcccgccc ctaactccgc ccagttccgc ccattctccg ccccatggct gactaatttt 7200
   ttttatttat gcagaggccg aggccgcctc ggcctctgag ctattccaga agtagtgagg 7260
   aggctttttt ggaggcctag gcttttgcaa agatcgatca agagacagga tgaggatcgt 7320
   ttcgcatgat tgaacaagat ggattgcacg caggttctcc ggccgcttgg gtggagaggc 7380
   tattcggcta tgactgggca caacagacaa tcggctgctc tgatgccgcc gtgttccggc 7440
   tgtcagcgca ggggcgcccg gttctttttg tcaagaccga cctgtccggt gccctgaatg 7500
   aactgcaaga cgaggcagcg cggctatcgt ggctggccac gacgggcgtt ccttgcgcag 7560
   ctgtgctcga cgttgtcact gaagcgggaa gggactggct gctattgggc gaagtgccgg 7620
   ggcaggatct cctgtcatct caccttgctc ctgccgagaa agtatccatc atggctgatg 7680
   caatgcggcg gctgcatacg cttgatccgg ctacctgccc attcgaccac caagcgaaac 7740
   atcgcatcga gcgagcacgt actcggatgg aagccggtct tgtcgatcag gatgatctgg 7800
   acgaagagca tcaggggctc gcgccagccg aactgttcgc caggctcaag gcgagcatgc 7860
   ccgacggcga ggatctcgtc gtgacccatg gcgatgcctg cttgccgaat atcatggtgg 7920
   aaaatggccg cttttctgga ttcatcgact gtggccggct gggtgtggcg gaccgctatc 7980
   aggacatagc gttggctacc cgtgatattg ctgaagagct tggcggcgaa tgggctgacc 8040
   gcttcctcgt gctttacggt atcgccgctc ccgattcgca gcgcatcgcc ttctatcgcc 8100
   ttcttgacga gttcttctga gcgggactct ggggttcgaa atgaccgacc aagcgacgcc 8160
   caacctgcca tcacgagatt tcgattccac cgccgccttc tatgaaaggt tgggcttcgg 8220
   aatcgttttc cgggacgccg gctggatgat cctccagcgc ggggatctca tgctggagtt 8280
   cttcgcccac cctaggggga ggctaactga aacacggaag gagacaatac cggaaggaac 8340
   ccgcgctatg acggcaataa aaagacagaa taaaacgcac ggtgttgggt cgtttgttca 8400
   taaacgcggg gttcggtccc agggctggca ctctgtcgat accccaccga gaccccattg 8460
   gggccaatac gcccgcgttt cttccttttc cccaccccac cccccaagtt cgggtgaagg 8520
   cccagggctc gcagccaacg tcggggcggc aggccctgcc atagcctcag gttactcata 8580
   tatactttag attgatttaa aacttcattt ttaatttaaa aggatctagg tgaagatcct 8640
   ttttgataat ctcatgacca aaatccctta acgtgagttt tcgttccact gagcgtcaga 8700
   ccccgtagaa aagatcaaag gatcttcttg agatcctttt tttctgcgcg taatctgctg 8760
   cttgcaaaca aaaaaaccac cgctaccagc ggtggtttgt ttgccggatc aagagctacc 8820
   aactcttttt ccgaaggtaa ctggcttcag cagagcgcag ataccaaata ctgtccttct   8880
   agtgtagccg tagttaggcc accacttcaa gaactctgta gcaccgccta catacctcgc   8940
   tctgctaatc ctgttaccag tggctgctgc cagtggcgat aagtcgtgtc ttaccgggtt   9000
   ggactcaaga cgatagttac cggataaggc gcagcggtcg ggctgaacgg ggggttcgtg   9060
   cacacagccc agcttggagc gaacgaccta caccgaactg agatacctac agcgtgagct   9120
   atgagaaagc gccacgcttc ccgaagggag aaaggcggac aggtatccgg taagcggcag   9180
   ggtcggaaca ggagagcgca cgagggagct tccaggggga aacgcctggt atctttatag   9240
   tcctgtcggg tttcgccacc tctgacttga gcgtcgattt ttgtgatgct cgtcaggggg   9300
   gcggagccta tggaaaaacg ccagcaacgc ggccttttta cggttcctgg ccttttgctg   9360
   gccttttgct cacatgttct ttcctgcgtt atcccctgat tctgtggata accgtattac   9420
   cgccatgcat                                                         9430

## Description

### TECHNICAL FIELD

The present invention relates to a new use of coccidia, specifically relates to the use of the coccidia as a live vector of vaccine.

### BACKGROUND ART

The recombinant vector vaccines developed by the use of transgenic technology based on pathogenic microorganisms, are attenuated on virulence but maintain strong cellular immune responses, so it has incomparable advantages over traditional vaccines. Current research is focusing on genetic recombinant vaccine and its mechanism of immunoprotection. Two aspects are required by an effective live vector vaccine: first, being safe to host; second, being able to effectively express and deliver the protective antigen.

At present, research-focused vaccine vectors comprise two categories: one includes attenuated viral vectors, such as poxvirus and adenovirus; the other contains enteric pathogenic attenuated bacteria and symbiotic bacteria, such as Salmonella and lactic acid bacteria. Live virus vector can induce effective immune response through displaying exogenous antigen on the host cell surface or releasing it into the extracellular environment to make it be recognized by the host immune system. It is possible for the enteric pathogenic attenuated bacteria and symbiotic bacteria after the genetic modification to sustainably and efficiently express autoantigens and exogenous antigens in the host body, thus effectively stimulating protective mucosal immunity, humoral and cellular immune responses against pathogens. However, there are inherent weaknesses for the attenuated viruses, enteric pathogenic bacteria and symbiotic bacteria as a live vaccine vector. As a vaccine vector, exogenous genetic fragments that can be integrated into viruses or bacteria are size-limited and it is difficult to carry out large-scale genetic modification. Viral vectors readily trigger host immune tolerance and are difficult to achieve the desired effect by oral administration. Enteric pathogens vectors such as Salmonella easily spread to other parts of the body in the host body, damaging other organs and tissues, and causing immune tolerance due to persistent infection; some salmonella strains are zoonotic pathogens, which can be potentially released into the environment and pose a potential threat to the health of human and animals. In addition, bacterial vectors can not perform glycosylation of the expressed exogenous protein and other modifications, thus causing lack of immune activity of the expressed exogenous protein.

The use of eukaryotes as a vehicle undoubtedly has a greater advantage over viruses and prokaryotes. A larger eukaryotic vector genome can be carried out large-scale genetic modification. And in eukaryotic cells, proteins can be correctly folded and modified and have normal activity. Although use of eukaryotic vectors faces greater challenge than viral and bacteria vectors, such research can be turned into reality from imagine since genomic and proteomic research has been relatively mature today.

Live oocyst vaccines, made of attenuated chicken coccidia, was available since half a century ago. At present its annual sales have been more than 300 million U.S. dollars, and it is playing an increasingly important role in the prevention and treatment of chicken coccidiosis. Now research on the interaction between the host and the live oocyst vaccine, as well as the mechanism of immune response against coccidiosis in the host has been in-depth.

The application US5976553 claims a transformed intracellular parasite of the order Eucoccodiorida, containing a DNA sequence exogenous to said parasite.

The review *article* Advances in Parasitology, 60 (2005), p.285-330 describes in general live-attenuated vaccines based on *Eimeria.*

The publication Molecufar & Biochemical Parasitology 153 (2007), p.213-215, describes a transient transfection of *Eimeria tenella* using fluorescent protein markers.

### SUMMARY OF INVENTION

The object of the present invention is to provide the application of coccidia as live vaccine vector;

The other object of the present invention is to provide a live coccidia vector vaccine. According to the present invention, target proteins expressed by live coccidia vector vaccine can induce humoral and cellular immune responses simultaneously and generate memory immune responses.

The target gene is cloned into the expression vector containing drug selection marker or fluorescent selection marker. Then the recombinant vector is introduced into the cells of coccidia via electroporation or other transfection methods. The transgenic coccidia expressing target protein or stably transfected coccidia containing expression vector that express exogenous protein can be obtained through drug selection or flow cytometry sorting. After inoculation into chicken or other poultry and mammals as live vaccines, transgenic coccidia or stably transfected coccidia express target proteins in the developing process, during which the temporospatial expression of target genes are regulated under the control of upstream and downstream sequences derived from relevant coccidia genes.

The coccidia vector in the present invention can be used to express many kinds of important antigenic proteins, such as *Eimeria maxima* TFP250 protein, spike glycoprotein such as hemagglutinin (HA) and other external and internal antigen protein of avian influenza virus (NA, NP, M1, M2, NS1, NS2, PA, PB1, and PB2 proteins) and Newcastle Disease virus F protein, all of which can induce host immune responses against pathogens.

Expression vectors for transecting coccidia can be any vectors that can express exogenous protein in coccidia. The recombinant vectors in the present invention include pH4sp-HA1-EYFP-ACTIN, pH4SP-M2e-EYFP-ACTIN, pH_{gra8}-E- HA-A3', pH_{gra8} -E- NA-A3', pH_{gra8}-E- NP-A3', pHDEA-TFP and so on. These vectors are used to transfect coccidia in order to obtain transgenic coccidia or stably transfected coccidia that can stably express the target protein. The obtained live coccidia vector vaccines are administrated orally to animals, inducing significant immune responses against both coccidia and viral infection.

Live vaccine in the present invention is based on precociously attenuated or wild-type coccidia, preferably *Eimeria, Wenyonella, Cryptosporidium* and so on. Chicken coccidia *Eimeria tenella* are the model species for transgenic research and live vaccine vector.

Coccidia have large size of genomic DNA, therefore there are a considerable number of sites that can be used for integration and also allow insertion of large fragments of exogenous gene. The exogenous protective antigen gene can be regulated by genetic flanking sequences of coccidia periodically in transcription stage and secreted into host cell using specific signal peptides of coccidia. Protein-secretory transgenic coccidia can stimulate strong humoral, cellular and mucosal immune responses in hosts.

Compared with bacterial and viral vector vaccines, transgenic coccidia vector have unique advantages. First, nuclear genome size of coccidia is more than 10Mb, which can accommodate large fragments of exogenous gene. Second, coccidia are eukaryotesand can express reactive antigen proteins modified by glycosylation. Third, the coccidia form parasitophorous vacuole within intestine epithelial cells, so its genetic fragments hold little probability to insert or integrate into the host cell genome. Fourth, due to the protection of oocyst wall, transgenic coccidia vaccine highly resist to acid environment of the crop and the stomach and can effectively reach specific sites of intestine. Fifth, oral vaccination via drinking water or feed is simple and applicable to poultry and mammal populations; and more importantly, coccidia oocysts can repeatedly infect hosts in the field, i.e, one immunization is equivalent to several times of vaccination. Sixth, coccidia have a short life cycle, and coccidia infection is self-limiting, so coccidia can be eliminated from the body after 3 generations, which does not cause immunotolerance problems. Seventh, different species of coccidia have strong host specificity without cross-species transmission, meanwhile, they widely distribute in fields. So releasing of transgenic coccidia in the field will not cause the spread of this kind of pathogen, hence live coccidia vector vaccines have a high bio-security.

The establishment of live vaccine vector of the present invention marks the birth of a new type of vaccine. In this invention, live coccidia oocysts express one or more (type) exogenous antigens thus can simultaneously stimulate humoral, cellular and mucosal immunity against a variety of pathogens. Transgenic coccidia vaccine are also useful complement to the eukaryotic expression system. Expressing target proteins of organisms in coccidia adds a new research direction for parasitology. The coccidia gene engineering vaccine based on present invention can be used in field for the control of coccidiosis in China. Transgenic coccidia vaccine can make survival rate, weight gain and feed conversion of the immune animal be higher than or equal to production performance of animals that use anticoccidial drugs, thus allowing the broad masses of farmers to benefit, and the said vaccine is pollution-free without residue.

### DESCRIPTION OF DRAWINGS

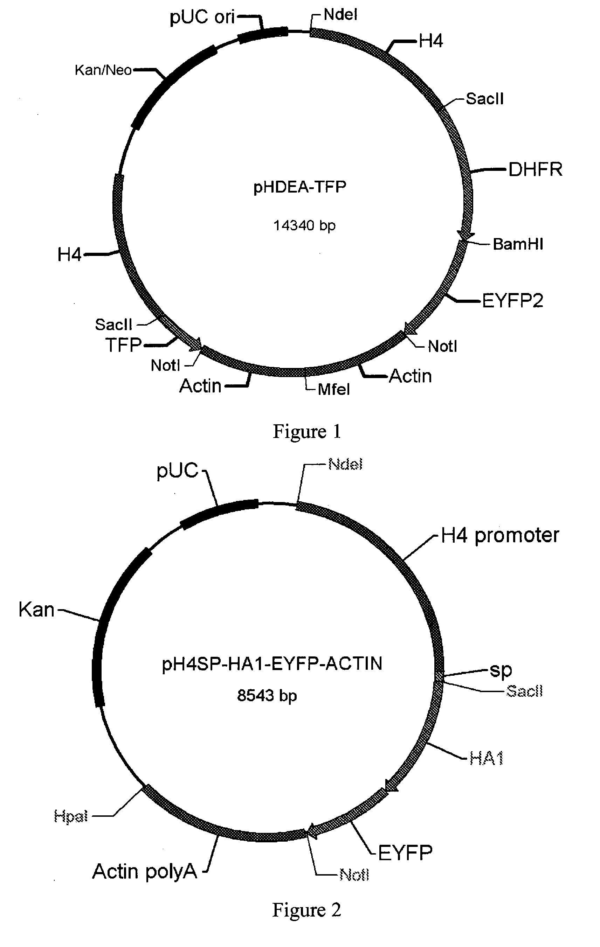

**Figure 1** is a constitutive schematic diagram of vector pHDEA-TFP, in which H4: *Eimeria tenella* histone 4 upstream regulatory sequence; DHFR: *Toxoplasma gondii* DHFR-TS gene coding region (for pyrimethamine resistance); EYFP2: Enhanced yellow-fluorescent protein gene coding region; Actin: Eimeria Actin gene downstream regulatory sequences. wherein, TFP is *Eimeria maxima* TFP250 genes, the insertion position of other antigen protein gene can be the same or different.
**Figure 2** is a constitutive schematic diagram of vector pH4sp-HA1-EYFP-ACTIN, in which, H4: *Eimeria tenella* histone 4 upstream regulatory sequence; SP: a signal peptide sequence of antigen 1 on the surface of *Eimeria tenella,* said sequence is 69 base pairs in length; HA1:H5N1 avian influenza virus HA antigen HA1 subunit; EYFP: enhanced yellow fluorescent protein gene coding region; Actin poly A: *Eimeria tenella* Actin gene downstream regulatory sequences.

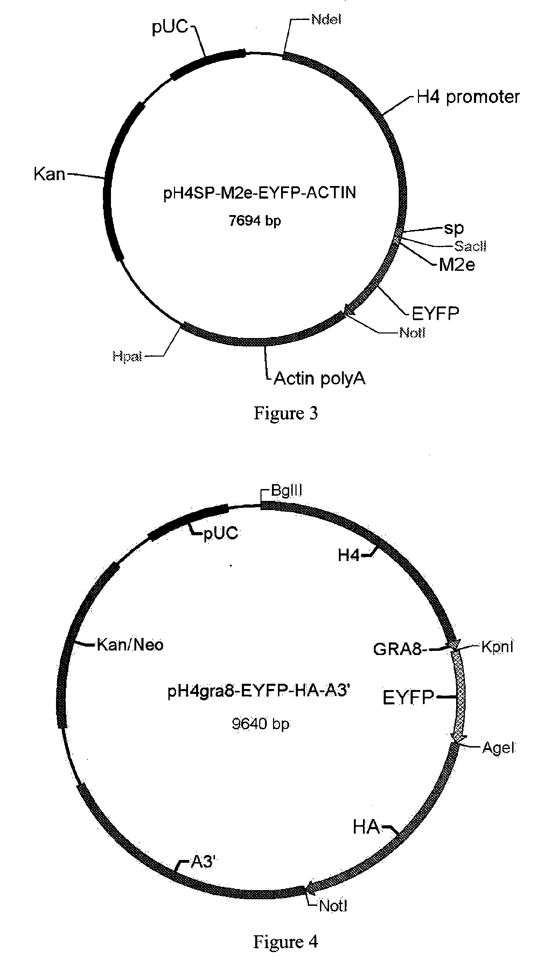

**Figure 3** is a constitutive schematic diagram of vector pH4SP-M2e-EYFP-ACTIN, in which, H4: *Eimeria tenella* histone 4 upstream regulatory sequence; SP: a signal peptide sequence of antigen 1 on the surface of *Eimeria tenella,* said sequence is 69 base pairs in length; M2e: extracellular domain of H5N1 avian influenza virus M2 antigen; EYFP: enhanced yellow fluorescent protein gene coding region; Actin poly A: *Eimeria tenella* Actin gene downstream regulatory sequences.
**Figure 4** is a constitutive schematic diagram of vector pHGRA8 - E-HA-A3', in which, H4: *Eimeria tenella* histone 4 upstream regulatory sequence; GRA8: *Toxoplasma gondii* dense granule protein GRA8 signal peptide coding sequence; EYFP: yellow fluorescent protein gene coding region; HA: the gene encoding area of H5N1 avian influenza virus HA protein; A 3': *Eimeria tenella* Actin gene downstream regulatory sequences.

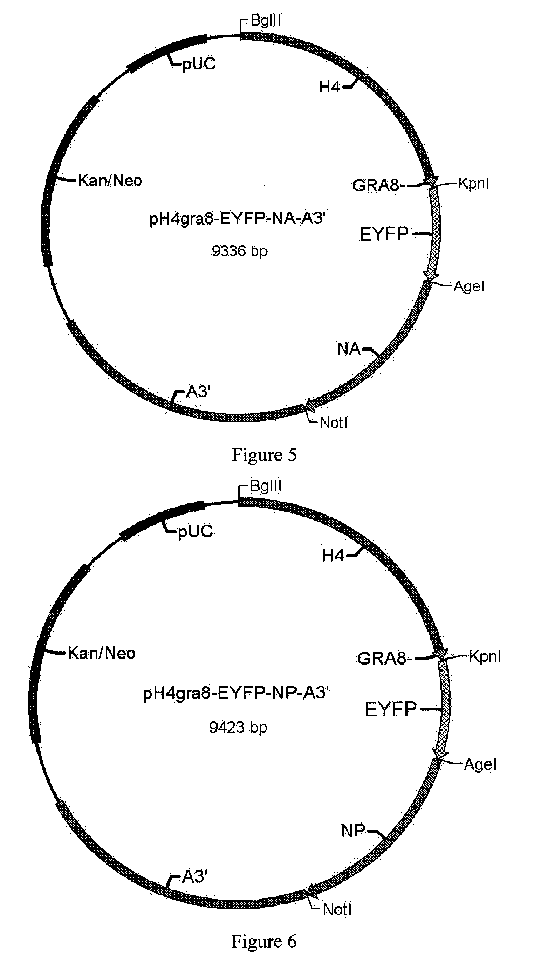

**Figure 5** is a constitutive schematic diagram of vector pHGRA8-E-NA-A3', in which, H4: *Eimeria tenella* histone 4 upstream regulatory sequence; GRA8: *Toxoplasma gondii* dense granule protein GRA8 signal peptide coding sequence; EYFP: yellow fluorescent protein gene coding region; NA: the gene encoding area of H5N1 avian influenza virus NA protein; A 3': *Eimeria tenella* Actin gene downstream regulatory sequences..
**Figure 6** is a constitutive schematic diagram of vector pHGRA8 - E-NP-A3 ', in which, H4: *Eimeria tenella* histone 4 upstream regulatory sequence; GRA8: *Toxoplasma gondii* dense granule protein GRA8 signal peptide coding sequence; EYFP: yellow fluorescent protein gene coding region; NP: gene coding area of H5N1 avian influenza virus NP protein; A 3': Ei*m*eria *tenella* Actin gene downstream regulatory sequences.

### SPECIFIC MODE OF CARRYING OUT THE INVENTION

The present invention will be further illustrated in combination with specific examples. It should be understood that these examples are only intended to explain the present invention without limiting the scope of protection of the invention.

### Example 1

The example is intended to use transgenic *Eimeria tenella* as a live vaccine vector to express the target gene, thus achieving simultaneous immunization of both the coccidia and certain pathogens.
*Eimeria maxima* TFP250 gene is taken as an example:

### 1. Constructing expression vector and cloning the target gene into the expression vector

10-day-old chickens are inoculated with *Eimeria tenella* strain BJ125 (Suo Xun, Qu Hong-fei, LIU Zong-quan, Lin Kun-hua, Lu Yan-li. Late-maturing strains breeding and immunogenicity study of Eimeria tenella. 1996, Journal of Agricultural Biotechnology 4 (2) ,78-79, in Chinese), after 6-9 days faeces or intestinal contents of infected chickens are collected to obtain coccidia oocysts, after sporulation, the sporocysts are extruded on slide glass, a sporocyst is sucked up using capillary pipette under the microscope, namely, a single sporocyst of *Eimeria tenella* isolated therefrom, the proliferated oocysts are collected after oral infection of the chicken with the isolated single sporocyst, that is, genetically identical coccidia line is obtained. After purification of oocysts, genome DNA is extracted with the general methods, *Eimeria tenella* gene regulatory sequence (histone 4 upstream regulatory sequences H4) is cloned using PCR method, *Eimeria maxima* TFP250 gene sequence is obtained using RT-PCR. The regulatory sequence H4, drug screening marker gene DHFR-TS, TFP250 and other components are constructed into the yellow fluorescence vector (purchased from BD Company), expression vector pHDEA-TFP required for *Eimeria tenella* transgene is constructed, the detailed contents are as follows:
Cloning *Toxoplasma gondii* DHFR-TS gene coding region: extracting total RNA of Toxoplasma gondii, amplifying and obtaining DHFR-TS gene using RT-PCR method, introducing restriction sites SacII and BamHI respectively at both ends, designing upstream and downstream primers (DHFR-UP: 5'-CATCCGCGGATGCAGAAACCGGTGTG-3'; DHFR-L:5'-CTGGATCCAAGACAGCCATCTCCATC-3'), obtaining DNA fragments with the size of 1.8 kb , recovering target fragment and ligating it into the vector PGEM-T, transforming and culturing overnight, extracting plasmids, identifying by enzyme digestion, introducing mutant site against pyrimethamine by PCR after DNA sequencing of the obtained candidate positive plasmids. Cloning *Eimeria tenella* histone 4 upstream regulatory sequences and *Eimeria tenella* Actin gene downstream regulatory sequences: using the coccidia genomic DNA as a template, primers at both ends introducing restriction site NdeI and SacII (H4-UP:5'-CAGCATATGAACCAGCAAAGGTAGCAAC-3';H4-L:5'-CTACCGCG GGATACCCTGGATGTTGTC-3') for amplification of H4 upstream regulatory sequence; primers at both ends of the yellow fluorescent protein gene and the downstream regulatory sequences introducing BamHI and MfeI sites, respectively designing primers (E2-UP : 5'-CATCCAGGGTATCGGATCCTGTCG-3'; Actin-L : 5'-CGCAATTGCTTCACATGGAACCCCTGG -3'), which are amplified by PCR to obtain a yellow fluorescent protein gene and the downstream regulatory sequences. Respectively obtaining upstream regulatory sequence with the size of 2.0kb and 2.9kb and the DNA fragments of the downstream regulatory sequences fused with the yellow fluorescent protein, recovering target fragment and ligating it with the vector PGEM-T, transforming and culturing overnight, extracting plasmids, identifying by enzyme digestion, carrying out DNA sequencing of the obtained candidate positive plasmids.
Cloning *Eimeria maxima* TFP250 gene: extracting the total RNA from Eimeria maxima and carrying out reverse transcription to obtain cDNA, during PCR amplication respectively introducing *Not* I and fused *Sac*II and *BamH*I sites (T-UP: 5'ATCCCGCGGGCCCGGGATCCTGTCGCCACCATGGAATTGCACCCCATTC CAG 3'; T-L: 5' ATTTGCGGCCGCCTACTGAATGTCGCCGCTGTCG 3') at both ends of TFP250 and performing amplication ,obtaining DNA fragments with the size of 660bp , recovering target fragment and ligating it with the vector PGEM-T ( purchased from Promega company), transforming after overnight, extracting plasmids, identifying by enzyme digestion, carrying out DNA sequcing for the obtained candidate positive plasmids. Ligating each digested gene with correspondingly digested yellow fluorescent vector, eventually obtaining the expression vector containing each component with its nucletide sequence as shown in sequence list SEQ ID NO.1.

### 2. Transfection and sorting of coccidia

The proliferated oocysts are collected after oral infection of the chicken with single oocysts of *Eimeria tenella,* 15-day-old chickens are inoculated with the above-mentioned oocysts, 200-500 oocysts for each chicken. The shed oocysts are collected and purified after 6 to 9 days, in this way continuous passages are done to get enough oocysts for the transfection.

By electroporation the constructed pHDEA-TFP is transfected into the sporozoites of *Eimeria tenella,* which are then inoculated in ileocecal opening of the chicken through cloaca, while pyrimethamine drug are added. Specifically, 1×10⁷ sporozoites are taken, 50µg of pHDEA-TFP plasmids are added, the voltage is 2kv, capacitance is 25, pulse is 0.3ms, after standing for 20min, the transfected sporozoite are inoculated again in ileocecal opening of the chicken through cloaca. After 24 hours of inoculation, the selected pressure is exerted, namely, pyrimethamine is added, the test is observed under fluorescence microscope.

Transgenic coccidia line or stably transfected coccidia line capable of stable expression can be obtained by repeating this process. Under fluorescence microscope it will be observed that transgenic coccidia capable of stable expression or the polypide of stably transfeced coccidia line can emit yellow fluorescence, luminescence rate is higher than 99%.

### 3. Inoculation into chickens

First, the oocysts are sporulated in vitro, the 1-5 day-old healthy chickens are orally inoculated with transgenic coccidia (precocious strain or wild-type strain), 200-500 /chicken. After 5 -7 days the clinical signs are tested, the number of oocysts per gram (OPG) is obtained and the lesion is scored.

### 4. The effect

After inoculation it is founded that the transgenic coccidia or stably transfected coccidia have potential immunogenicity against *Eimeria tenella* and *Eimeria maxima*, or other pathogenic infections,

Seven-day-old AA broiler chickens are inoculated with the oocysts of transgenic coccidia , 1.0 × 10³ oocyst/chicken. Fourteenth days after inoculation, the number of oocysts releaesed from each chicken was significantly lower than those non-immunized but challenged birds (P <0.05), and there is no significant difference between the immunizing-challenging group and the group immunized with same dose oocysts of parent strain (P> 0.05). It indicates that inoculation with the transgenic coccidia, 1.0 × 10³ oocyst/chicken, can provide seven-day-old chicks with immunoprotection. Another immune procedure is to use low-dose immunization of transgenic coccidia oocysts, 200 oocysts /chicken, to immunize 4-day-old AA broiler chickens, at the fourteenth day after immunizing two times, the chickens are challenged with transgenic coccidia or parent strain oocysts, 1.0 × 10³ oocyst/chicken, only to find the minimal amount of fecal oocysts are discharged along with Faeces, the oocyst production per chicken is significantly lower than those in non-immunized groups without attacking-coccidia (P <0.01), similar with the groups immunized with parent strains, the chickens in the group immunized with the transgenic coccidiatwice are completely protected. The data from two kinds of immune schemes indicate that the expression of exogenous genes does not affect the immunogenicity of the transgenic coccidia. After inoculation, the pathological grading, oocyst production of coccidia in the infected chickens and cecal lesion score all decrease, the decline of weight gain of chickens is improved, OPG value is 6 × 10⁴-6 × 10⁶, a good immune protection is generated to prevent lethal dose of coccidia attack or the challenged infections induced by other pathogens.

### Example 2

pH4sp-HA1-EYFP-ACTIN, pH4SP-M2e-EYFP-ACTIN, pH_{gra8}-E- HA-A3', pH_{gra8} -E-NA-A3' and pH_{gra8}-E-NP-A3' expression vector are constructed respectively according to the method similar to the above-mentioned methods, which are then transfected into the coccidia, followed by obtaining the coccidia that can stably express the target gene through screening. Cohabitation infection test indicates that the obtained transgenic coccidia have high protective rate against coccidia infection and virus infection (>90%).

### INDUSTRIAL APPLICABILITY

The present invention provides a new use of coccidia, specifically relates to the use of coccidia as a live vaccine vector. The present invention further provides a live vaccine using transgenic coccidia as a vector, which is capable of expressing exogenous protein. The present live coccidia vector vaccine can induce humoral, cellular and mucosal immune responses simultaneously, and generate memory responses. This invention can be readily carried out and has stable effect and high biological safety without generating immune tolerance. The coccidia gene engineering vaccine based on present invention can be used in field for the control of coccidiosis in China. Transgenic coccidia vaccine can make survival rate, weight gain and feed conversion of the immune animal be higher than or equal to production performance of animals that use anticoccidial drugs, thus allowing the broad masses of farmers to benefit, and the said vaccine is pollution-free without residue.

The description of the sequences:
SEQ ID NO.1~6 are in sequence: full sequences of expression vector pHDEA-TFP, pH4sp-HA1-EYFP-ACTIN, pH4SP-M2e-EYFP-ACTIN, pH_{gra8}-EYFP-HA-A3', pH_{gra8}-EYFP- NA-A3' and pH_{gra8}-EYFP- NP-A3'.

## Claims

1. A coccidia live vaccine expressing target proteins as immune live vector, said live vaccine being a stably transfected coccidia that contains, an expression vector and can express target proteins, in which said expression vector is selected from:
pH4sp-HA1-EYFP-ACTIN (SEQ. ID NO. 2),
pH4SP-M2e-EYFP-ACTIN (SEQ. ID NO. 3),
pHgra8--EYFP- HA-A3' (SEQ. ID NO. 4),
pH_{gra8}- -EYFP- NA-A3' (SEQ. ID NO. 5),
pH_{gra8}--EYFP-NP-A3' (SEQ. ID NO. 6), or
pHDEA-TFP (SEQ. ID NO. 1),
wherein the coccidia is of the family Eimeriidae.

2. The live vaccine as claimed in claim 1, in which said expression vector is site-specific integrated or randomly integrated into the coccidia genome thereof and can temporally regulate the expression of exogenous proteins.

3. The live coccidian vaccines as claimed in any one of claims 1 to 2, in which said target proteins are antigen proteins of pathogens.

4. The live vaccine as claimed in claim 3, **characterized in that** said antigen proteins of pathogens are selected from avian influenza virus proteins including HA, NA, NP, M1, M2, NS1, NS2, PA, PB1 and PB2, F protein of Newcastle Disease virus or TFP250 protein of *Eimeria maxima.*

5. The live vaccine as claimed in any one of claims 1 to 4, in which said coccidia are members of the family Eimeriidae or the family Cryptosporidiidae.

6. Use of coccidia in the preparation of the live vector vaccines described in claim 1.

7. The use as claimed in claim 6, in which said coccidia are members of the family Eimeriidae or the family Cryptosporidiidae.

8. The use as claimed in claim 7, **characterized in that** said coccidia are Eimeriidae coccidia or Cryptosporidiidae coccidia in poultry or mammals.

## Patentansprüche

1. Kokzidien-Lebendimpfstoff, der Zielproteine als lebenden Immunvektor exprimiert, wobei der Lebendimpfstoff stabil transfizierte Kokzidien sind, die einen Expressionsvektor enthalten und Zielproteine exprimieren können, wobei der Expressionsvektor ausgewählt ist aus:
pH4sp-HA1-EYFP-ACTIN (SEQ ID NO. 2),
pH4SP-M2e-EYFP-ACTIN (SEQ ID NO. 3),
pHgra8--EYFP-HA-A3' (SEQ ID NO. 4),
pHgra8--EYFP-NA-A3' (SEQ ID NO. 5),
pHgra8--EYFP-NP-A3' (SEQ ID NO. 6) oder
pHDEA-TFP (SEQ ID NO. 1),
wobei die Kokzidien zur Familie der Eimeriidae gehören.

2. Lebendimpfstoff nach Anspruch 1, wobei der Expressionsvektor ortsspezifisch oder willkürlich in das Kokzidiengenom integriert ist und die Expression von exogenen Proteinen zeitlich regeln kann.

3. Kokzidien-Lebendimpfstoffe nach einem der Ansprüche 1 bis 2, wobei die Zielproteine Antigenproteine von Pathogenen sind.

4. Lebendimpfstoff nach Anspruch 3, **dadurch gekennzeichnet, dass** die Antigenproteine von Pathogenen aus Vogelgrippevirusproteinen, einschließlich HA, NA, NP, M1, M2, NS1, NS2, PA, PB1 und PB2, F-Protein des Newcastle-Krankheits-Virus oder TFP250-Protein von Eimeria maxima, ausgewählt sind.

5. Lebendimpfstoff nach einem der Ansprüche 1 bis 4, wobei die Kokzidien Mitglieder der Familie Eimeriidae oder der Familie Cryptosporidiidae sind.

6. Verwendung von Kokzidien zur Herstellung der Lebend- Vektorimpfstoffe nach Anspruch 1.

7. Verwendung nach Anspruch 6, wobei die Kokzidien Mitglieder der Familie Eimeriidae oder der Familie Cryptosporidiidae sind.

8. Verwendung nach Anspruch 7, **dadurch gekennzeichnet, dass** die Kokzidien Eimeriidae coccidia oder Cryptosporidiidae coccidia in Geflügel oder Säugetieren sind.

## Revendications

1. Vaccin vivant contre la coccidiose exprimant des protéines cibles en tant que vecteur vivant immunitaire, ledit vaccin vivant étant une coccidie transfectée de manière stable qui contient un vecteur d'expression et est capable d'exprimer des protéines cibles, dans lequel ledit vecteur d'expression est sélectionné parmi :
pH4sp-HA1-EYFP-ACTIN (SEQ. ID NO. 2),
pH4SP-M2e-EYFP-ACTIN (SEQ. ID NO. 3),
pHgra8--EYFP- HA-A3' (SEQ. ID NO. 4),
pHgra8- -EYFP- NA-A3' (SEQ. ID NO. 5),
pHgra8-EYFP-NP-A3' (SEQ. ID NO. 6), ou
pHDEA-TFP (SEQ. ID NO. 1),
dans lequel la coccidie est de la famille des Eimeriidae. '

2. Vaccin vivant selon la revendication 1, dans lequel ledit vecteur d'expression est intégré de manière spécifique de site ou intégré de manière aléatoire dans le génome des coccidies de ce dernier et peut temporairement réguler l'expression de protéines exogènes.

3. Vaccins vivants contre la coccidiose selon l'une quelconque des revendications 1 à 2, dans lequel lesdites protéines cibles sont des protéines antigènes de pathogènes.

4. Vaccin vivant selon la revendication 3, **caractérisé en ce que** lesdites protéines antigènes de pathogènes sont sélectionnées parmi des protéines du virus de la grippe aviaire incluant HA, NA, NP, M1, M2, NS1, NS2, PA, PB1 et PB2, la protéine F du virus de la maladie de Newcastle ou la protéine TFP250 de *Eimeria maxima.*

5. Vaccin vivant selon l'une quelconque des revendications 1 à 4, dans lequel lesdites coccidies sont de la famille des Eimeriidae ou de la famille des Cryptosporidiidae.

6. Utilisation de coccidies dans la préparation des vaccins-vecteurs vivants décrits dans la revendication 1.

7. Utilisation selon la revendication 6, dans laquelle lesdites coccidies sont de la famille des Eimeriidae ou de la famille des Cryptosporidiidae.

8. Utilisation selon la revendication 7, **caractérisée en ce que** lesdites coccidies sont des coccidies Eimeriidae ou des coccidies Cryptosporidiidae chez les volailles ou les mammifères.
